# EUROPEAN PATENT SPECIFICATION

(11) **EP 3 077 048 B1**
(45) Date of publication and mention of the grant of the patent: **17.07.2019**
(21) Application number: 14868378.2
(22) Date of filing: 08.12.2014
(51) Int. Cl.: A61P 7/02, A61K 39/395, A61K 38/00, G01N 33/53, C07K 16/28, C07K 16/24, A61K 38/17, C07K 16/18, G01N 33/68

(54) **COMPOSITIONS AND METHODS OF TREATING THROMBOSIS**
ZUSAMMENSETZUNGEN UND VERFAHREN ZUR BEHANDLUNG VON THROMBOSE
COMPOSITIONS ET MÉTHODES DE TRAITEMENT DE THROMBOSE

(30) Priority: 07.12.2013 US 201361913263 P
(43) Date of publication of application: 12.10.2016
(73) Proprietor: Case Western Reserve University, Cleveland OH 44116 (US)
(72) Inventor: SIMON, Daniel I, Moreland Hills, Ohio 44022 (US)
(74) Representative: Maiwald Patent- und Rechtsanwaltsgesellschaft mbH
(86) International application number: PCT/US2014/069134
(87) International publication number: WO 2015/085311

(56) References cited:
- WO-A1-93/06848
- WO-A1-2006/122723
- WO-A2-03/069349
- US-A- 6 090 367
- US-A1- 2008 219 998
- US-A1- 2009 246 810
- YUNMEI WANG ET AL: "Platelet-derived S100 family member myeloid-related protein-14 regulates thrombosis", JOURNAL OF CLINICAL INVESTIGATION, vol. 124, no. 5, 1 May 2014 (2014-05-01), pages 2160-2171, XP055386570, US ISSN: 0021-9738, DOI: 10.1172/JCI70966
- KEIKO YONEKAWA ET AL: "Myeloid related proteins activate Toll-like receptor 4 in human acute coronary syndromes", ATHEROSCLEROSIS, ELSEVIER, AMSTERDAM, NL, vol. 218, no. 2, 8 June 2011 (2011-06-08), pages 486-492, XP028301383, ISSN: 0021-9150, DOI: 10.1016/J.ATHEROSCLEROSIS.2011.06.020 [retrieved on 2011-06-17]
- HEALY ET AL.: 'Platelet Expression Profiling and Clinical Validation of Myeloid-Related Protein-14 as a Novel Determinant of Cardiovascular Events'.' CIRCULATION vol. 113, no. 19, 16 May 2006, pages 2278 - 2284, XP002401377
- KERKHOFF ET AL.: 'Interaction of S 100A8 /S 100A9 -Arachidonic Acid Complexes with the Scavenger Receptor CD 36 May Facilitate Fatty Acid Uptake by Endothelial Cells' BIOCHEMISTRY vol. 40, no. 1, 09 January 2001, pages 241 - 248, XP055348376

## Description

### TECHNICAL FIELD

This application relates to compositions and methods for preventing and/or treating hypercoagulation and/or thrombosis in a subject in need thereof and to methods of predicting cardiovascular disease activity in a subject.

### BACKGROUND

Thrombotic complications are a major cause of death in the industrialized world. Examples of these complications include acute myocardial infarction, unstable angina, chronic stable angina, transient ischemic attacks, strokes, peripheral vascular disease, preeclampsia/eclampsia, deep venous thrombosis, embolism, disseminated intravascular coagulation and thrombotic cytopenic purpura. Thrombotic and restenotic complications also occur following invasive procedures, e.g., angioplasty, carotid endarterectomy, post CABG (coronary artery bypass graft) surgery, vascular graft surgery, stent placements and insertion of endovascular devices and prostheses. It is generally thought that platelet aggregates play a critical role in these events. Blood platelets, which normally circulate freely in the vasculature, become activated and aggregate to form a thrombus with disturbed blood flow caused by ruptured atherosclerotic lesions or by invasive treatments such as angioplasty, resulting in vascular occlusion. Platelet activation can be initiated by a variety of agents, e.g., exposed subendothelial matrix molecules such as collagen, or by thrombus, which is formed in the coagulation cascade. US 2009/246810 A1 discloses a method for determining the risk whether an individual showing at least one symptom of an evolving acute coronary syndrome (ACS) is suffering from an acute coronary syndrome comprising the steps of (a) measuring the level of MRP 8/14, wherein (b) if the level of MRP 8/14 is at least increased, then the individual is at risk of suffering from an acute coronary syndrome. Y. Wang et al., Journal of Clinical Investigation, 2014, vol. 124, no. 5, pages 2160 to 2171 relates to the expression of the gene encoding the S100 calcium-modulated protein family member MRP-14 also known as S100A9 is elevated in platelets from patients presenting with acute myocardial infarction compared with those of patients presenting with acute myocardial infarction compared with those from patients with stable coronary artery disease.

### SUMMARY

The present invention is directed to a composition for use in inhibiting thrombosis in a subject having or at risk of thrombosis, the composition comprising an agent effective to inhibit MRP-8/14 binding to platelet CD36 and inhibit thrombosis in the subject, wherein the agent binds to the MRP-8/14 heterodimer and inhibits MRP-8/14 induced activation of platelet CD36 signaling and/or thrombosis formation in the subject, wherein the agent comprises an anti-MRP-14 antibody or antigen binding fragment thereof that inhibits binding of MRP-8/14 to CD36. In a preferred embodiment, the agent inhibits thrombosis in the subject without inhibiting hemostasis.

### BRIEF DESCRIPTION OF THE DRAWINGS

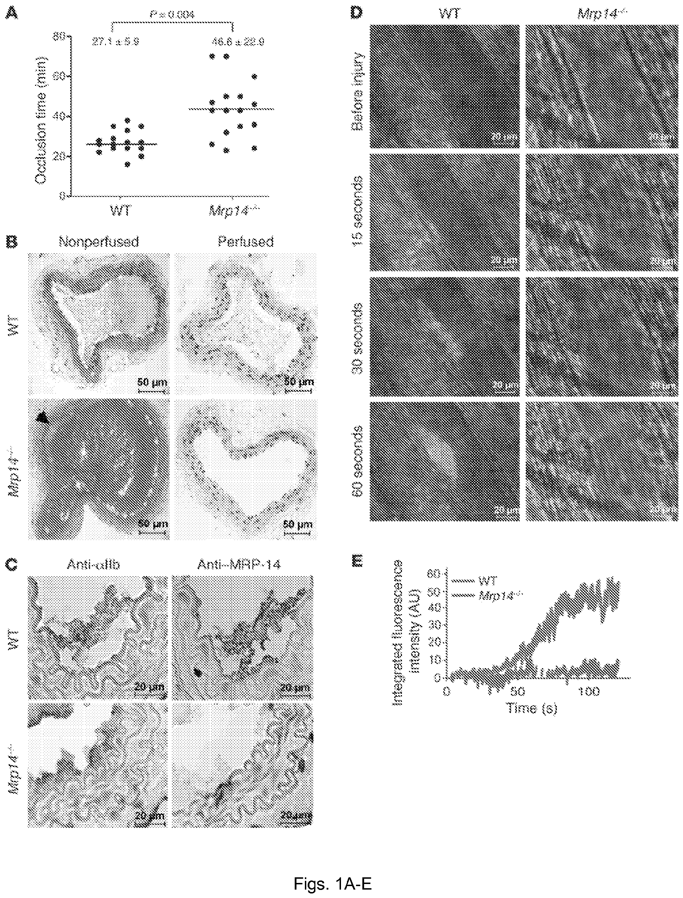

Figs. 1(A-E) illustrate plots and images showing MRP-14 deficiency prolongs thrombotic occlusion time. (A) Occlusion times following photochemical injury of the carotid artery in WT and *MRP14*^{*-*/*-*} mice (mean ± SD, n = 16 per group). (B) Histologic sections of nonperfused and perfusion-fixed carotid arteries 25 minutes after injury demonstrating occlusive thrombus in WT arteries and patency in *Mrp14*^{*-*/*-*} arteries. (C) Immunohistochemistry of MRP-14 and the platelet-specific marker GPIIb after carotid artery photochemical injury. (D) Thrombus formation *in vivo* after laser-induced injury to the arteriolar wall in the cremaster microcirculation of *Mrp14*^{*-*/*-*} mice was compared with that of WT mice using intravital microscopy. Platelets were labeled *in vivo* using an FITC-conjugated rat anti-mouse CD41 antibody. Representative intravital images at indicated times following laser pulse. Scale bars: 50 µm (B) and 20 µm (C and D). (E) Continuous, real-time thrombosis profiles of one representative experiment (n = 10-15 arterioles per group).

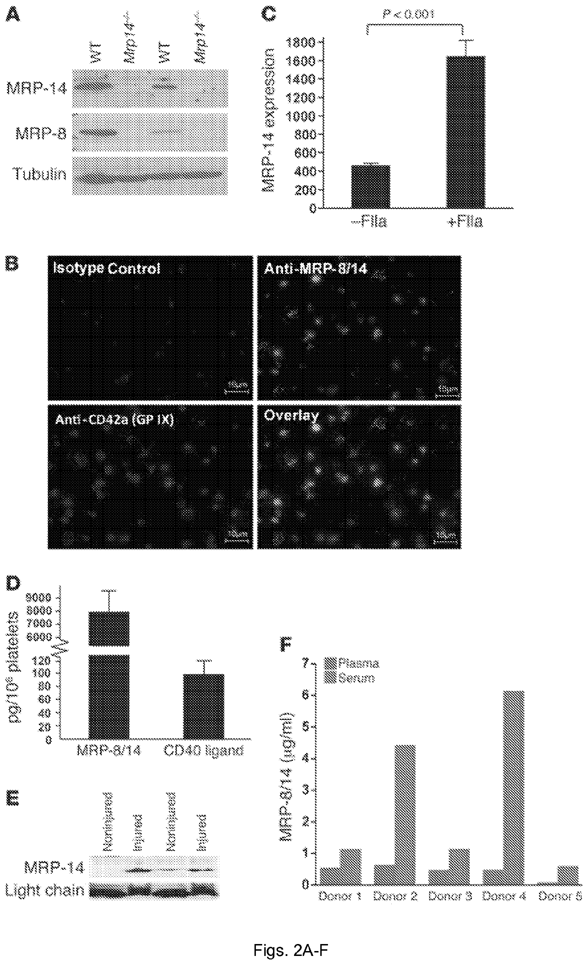

Figs. 2(A-F) illustrate immunoassays, graphs, and images showing platelets express MRP-8 and MRP-14. (A) MRP-8 (8 kDa) and MRP-14 (14 kDa) protein expression was examined in gel-filtered (lanes 1 and 2) and washed (lanes 3 and 4) WT and *Mrp14*^{*-*/*-*} platelets. Platelets were lysed with SDSPAGE- reduced sample buffer and then immunoblotted sequentially with anti-MRP-8, anti-MRP-14, and anti-tubulin antibodies. (B) Expression of MRP-8/14 in human platelets adherent to fibrinogen-coated cover-slips was investigated using combined immunofluorescence with one antibody that recognizes the MRP-8/14 complex and another the platelet-specific marker GPIX. Robust MRP-8/14 staining was evident in GPIX-positive human platelets. Scale bars: 10 µm. (C) Platelet surface expression of MRP-14 in resting and thrombin-stimulated (FIIa-stimulated) platelets was determined by flow cytometry (mean fluorescence intensity; n = 4 per group). (D) MRP-8/14 and CD40L protein content in gel-filtered human platelets (mean ± SD, n = 5 normal human donors). (E) Immunoblot of plasma (18 µl per lane) from non-injured animals and from animals 10 minutes after photochemical carotid artery injury using anti-MRP-14 antibody. Equal loading was verified by IgG light chain. (F) Paired plasma and serum levels of MRP-8/14 (µg/ml) from 5 normal human donors.

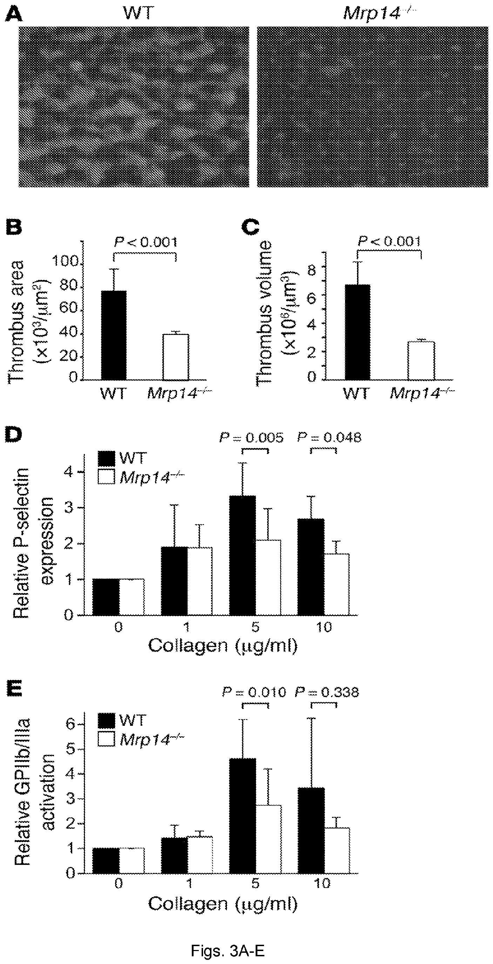

Figs. 3(A-E) illustrate an image and graphs showing MRP-14 deficiency attenuates thrombus formation under flow and is associated with defects in collagen-induced platelet activation. (A) Platelet thrombi on collagen-coated capillaries following perfusion of rhodamine 6G-labeled blood from WT and *Mrp14*^{*-*/*-*} mice at an arterial shear rate of 625 s-1. Original magnification, ×40; observation area, 360 × 270 mm. Thrombus formation (B, area; C, volume) was quantified using computer-assisted imaging analysis (n = 3-5 per group). Flow cytometric analysis of P-selectin expression (D) and assessment of GPIIb/IIIa activation using staining with the JON/A antibody (E) following stimulation of washed platelets from WT (black bars) and *Mrp14*^{*-*/*-*} (white bars) mice with 0 to 10 µg/ml collagen (n = 5 per group).

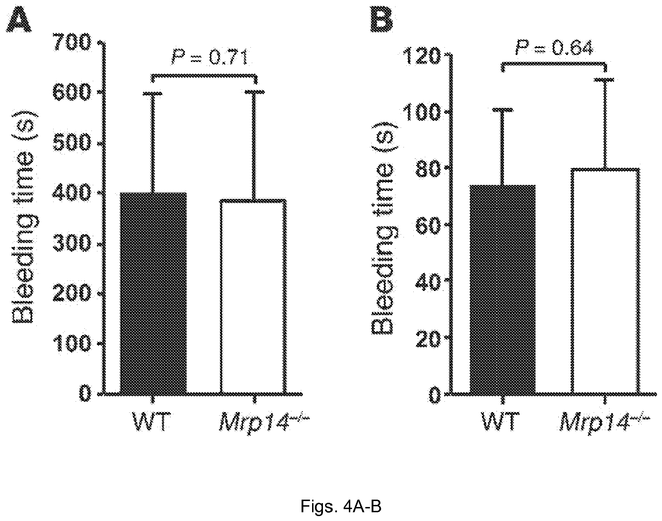

Figs. 4(A-B) illustrate graphs showing MRP-14 deficiency has no effect on tail bleeding time. To assess the role of MRP-14 in hemostasis, tail bleeding times were assessed in WT and *Mrp14*^{*-*/*-*} mice using complete cessation of bleeding either for 3 minutes (A) or 30 seconds (B) as the criterion for determination of bleeding time (mean ± SD, *n* = 16 per group).

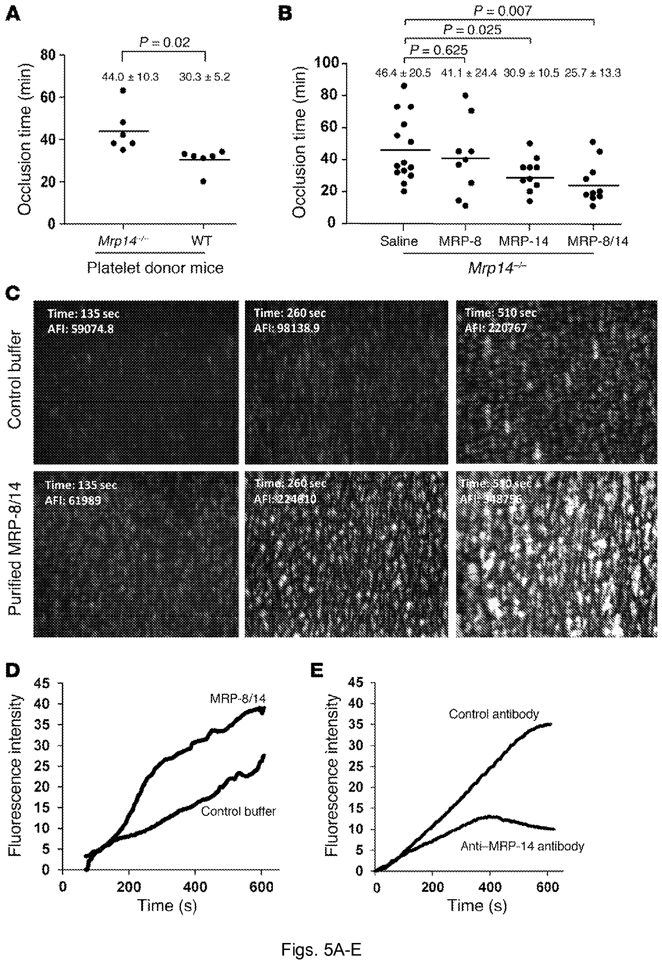

Figs. 5(A-E) illustrate plots and images showing transfusion of WT platelets or infusion of purified MRP-14 or MRP-8/14 shortens the prolonged time to carotid artery occlusion in *Mrp14*^{*-*/*-*}mice. (A) Transfusion of WT or *Mrp14*^{*-*/*-*} donor gel-filtered platelets (1 × 10⁸ in 250 µl) into *Mrp14*^{*-*/*-*} recipient mice prior to carotid artery photochemical injury. Occlusion times following injury for *Mrp14*^{*-*/*-*} recipient mice receiving WT donor or *Mrp14*^{*-*/-} donor platelets (mean ± SD, *n* = 6 per group). (B) Thrombotic occlusion time with intravenous infusion of saline *(n* = 14), purified human MRP-8 (0.08 µg/g mouse; *n* = 9), MRP-14 (0.08 µg/g mouse, *n* = 10), or MRP-8/14 (0.08 µg/g mouse; *n* = 10) into *Mrp14*^{*-*/*-*} recipient mice prior to carotid artery photochemical injury. (C) Exogenous MRP-8/14 enhanced platelet thrombus formation on collagen under flow. Platelet thrombi on collagen-coated capillaries following perfusion (shear rate of 625 s-1) of rhodamine 6G-labeled blood from *Mrp14*^{*-*/*-*} blood that was treated with purified human MRP-8/14 (5 µg/ml) or control buffer. Original magnification, ×40; observation area, 360 × 270 mm. (D) Continuous, real-time thrombosis profiles of one representative experiment. (E) Platelet thrombus formation under flow in anticoagulated human whole blood was evaluated in the presence of the anti-MRP-14 monoclonal antibody 1H9 versus control antibody (10 µg/ml). Continuous, real-time thrombosis profiles of one representative experiment.

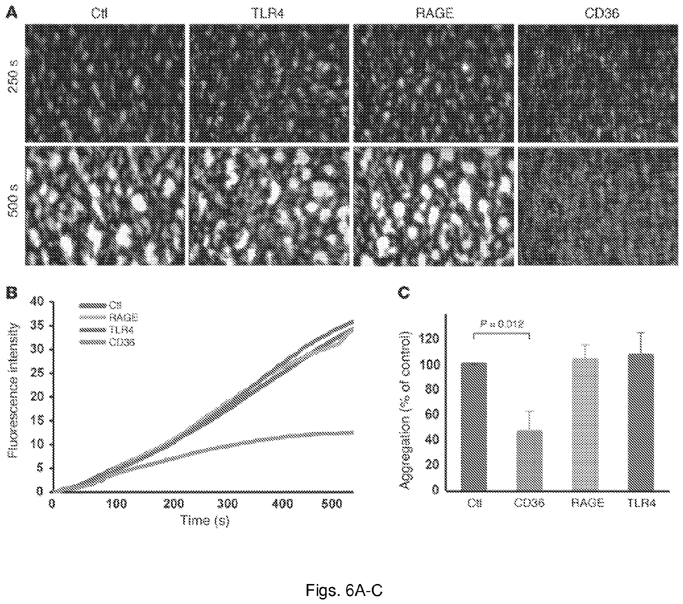

Figs. 6(A-C) illustrate an image, plot, and graph showing CD36 is a putative receptor for MRP-14. (A) Platelet thrombus formation under flow in anticoagulated human whole blood was evaluated in the presence of blocking monoclonal antibodies (10 µg/ml) against CD36, RAGE, TLR4, or control IgG. Original magnification, ×40; observation area, 360 × 270 mm. (B) Continuous, real-time thrombosis profiles of platelet thrombus formation under flow quantified (C) as a percentage of aggregation relative to control antibody (*n* = 3-5 per antibody).

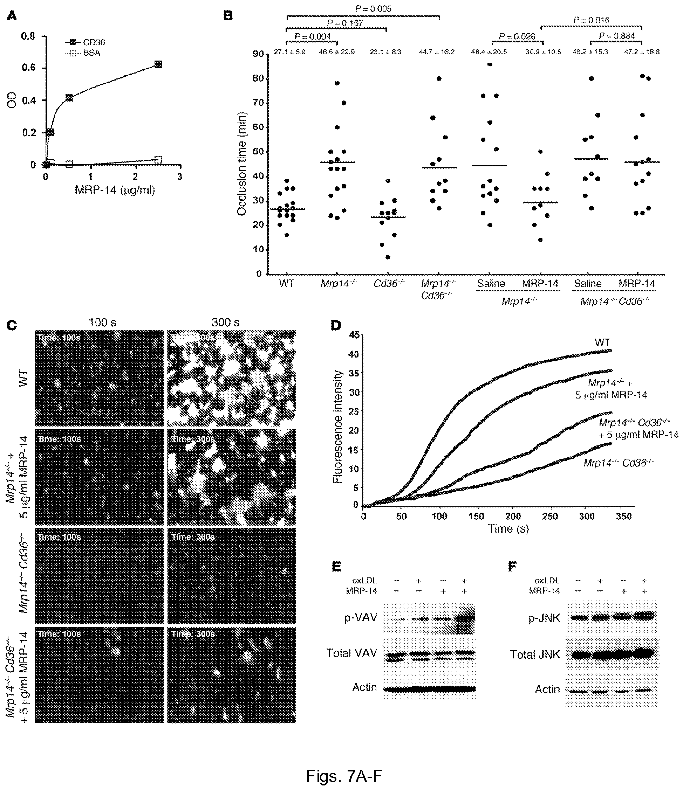

Figs. 7(A-F) illustrate plots, images, and immunoassays showing CD36 is required for MRP-14 action. (A) Binding of purified MRP-14 (0-2.5 µg/ml) to purified soluble CD36-coated or BSA-coated wells. (B) Thrombotic occlusion time after carotid artery photochemical injury in indicated mouse strains and occlusion time with intravenous infusion of saline or purified human MRP-14 (0.08 µg/g mouse) into *Mrp14*^{*-*/*-*} or *Mrp14*^{*-*/}*⁻ Cd36*^{*-*/*-*} recipient mice prior to photochemical injury. (C) Purified MRP-14 restores platelet thrombus formation under flow in *Mrp14*^{*-*/*-*}, but not *Mrp14*^{*-*/}*⁻ Cd36*^{*-*/*-*}, murine whole blood. Platelet thrombi on collagen coated capillaries following perfusion (shear rate of 625 s-1) of rhodamine 6G-labeled *Mrp14*^{*-*/*-*} blood from WT, *Mrp14*^{*-*/*-*}, or *Mrp14*^{*-*/}*⁻ Cd36*^{*-*/*-*} mice that was treated with purified human MRP-14 (5 µg/ml) or control buffer. Original magnification, ×40; observation area, 360 × 270 mm. (D) Continuous, real-time thrombosis profiles of the average fluorescence of three independent experiments. MRP-14 induced phosphorylation of VAV (E) and JNK (F) in platelets. Gel-filtered human platelets (2 × 10⁸/ml) containing 2 mM CaCl2 and 1 mM MgCl2 were incubated with 50 µg/ml oxLDL, 1 µg/ml MRP-14, or a combination of these for 10 minutes, and platelet lysates were analyzed by immunoblotting with anti-phosphoprotein antibodies. The membranes were then stripped and reprobed with antibodies against the total relevant protein and actin. Results are representative of three independent experiments from different donors.

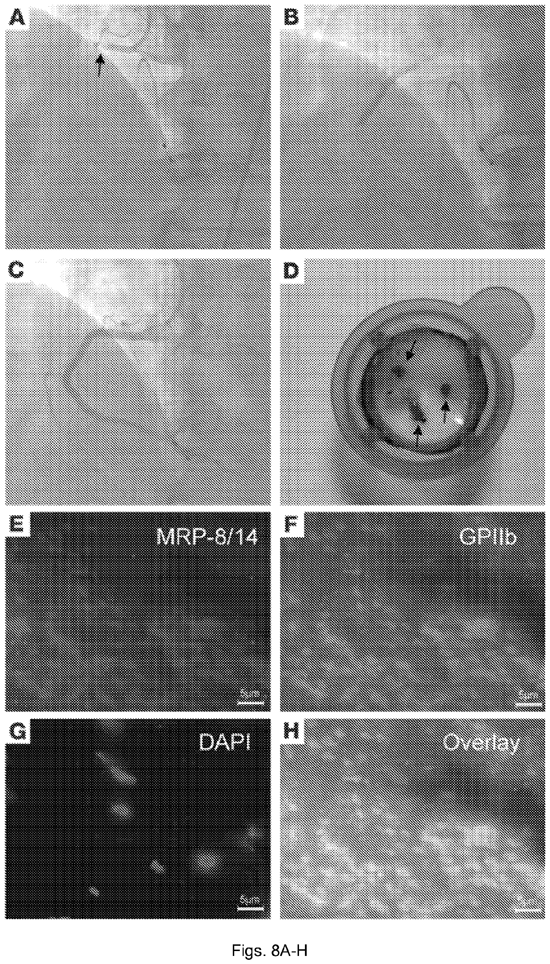

Figs. 8(A-H) illustrate images showing platelet expression of MRP-8/14 in human coronary artery thrombus. Coronary artery thrombus was obtained from a patient presenting to the cardiac catheterization laboratory with acute inferior STEMI. (A) Angiogram demonstrating thrombotic occlusion (arrow) of the proximal right coronary artery. (B) Deployment of stent at the site of the right coronary artery occlusion. (C) Final angiogram showing a widely patent right coronary artery with no significant luminal narrowing. (D) Thrombectomy catheter aspirate after passage through filter shows coronary artery thrombi (arrows). Immunofluorescence staining of coronary artery thrombus with anti-MRP-8/14 (E) and antiplatelet GPIIb (F) antibodies. Nuclei are stained with DAPI. (G) Colocalization of MRP-8/14 and platelets are depicted in the overlay (H). Scale bars: 5 µm (E-H).

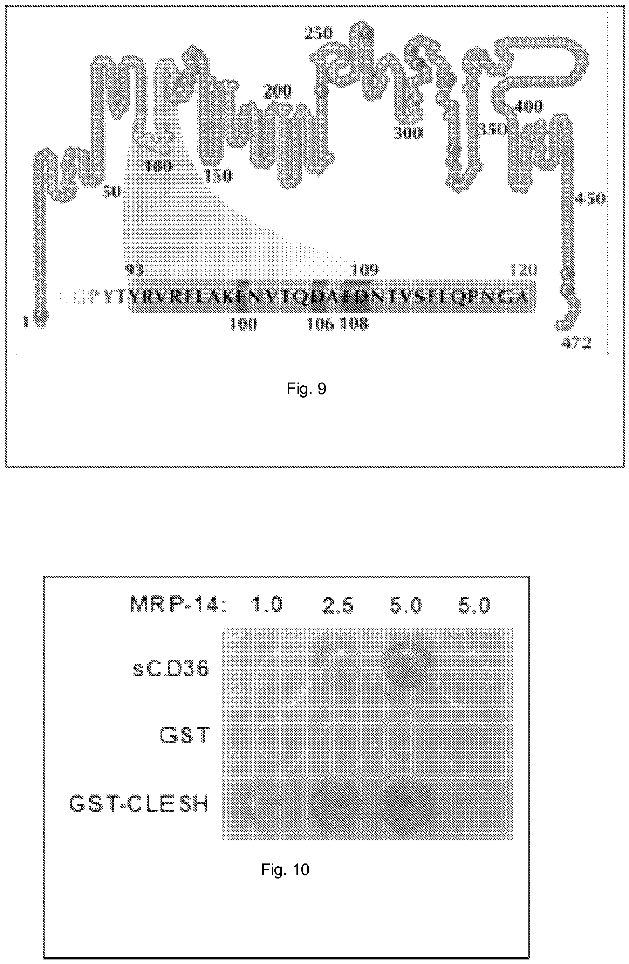

Fig. 9 is a schematic illustration of the secondary structure of CD36 modeled using the Chou Fosman predictive algorithm. CLESH domain (aa93-120) (SEQ ID NO: 3) that mediates binding of TSP-1 and MRP-14 is highlighted along with the acidic cluster for TSP-1 interaction.
Fig. 10 is an image of a plate binding assay that shows MRP-14 binds to CD36 CLESH domain. The plate binding assay shows binding of MRP14 (1-5 µg/ml) to sCD36, GST-CLESH, or GST-control.

### DETAILED DESCRIPTION

Methods involving conventional molecular biology techniques are described herein. Such techniques are generally known in the art and are described in detail in methodology treatises, such as Current Protocols in Molecular Biology, ed. Ausubel et al., Greene Publishing and Wiley-Interscience, New York, 1992 (with periodic updates). Unless otherwise defined, all technical terms used herein have the same meaning as commonly understood by one of ordinary skill in the art to which the application pertains. Commonly understood definitions of molecular biology terms can be found in, for example, Rieger et al., Glossary of Genetics: Classical and Molecular, 5th Edition, Springer-Verlag: New York, 1991, and Lewin, Genes V, Oxford University Press: New York, 1994.

The term "comprising" is intended to mean that the compositions and methods include the recited elements, but do not exclude other elements. "Consisting essentially of', when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of' shall mean excluding more than trace elements of other ingredients and substantial method steps for administering the compositions. Embodiments defined by each of these transition terms are within the scope of this invention.

The term "agent" is used herein to denote a chemical compound, a mixture of chemical compounds, a biological macromolecule, or an extract made from biological materials.

The terms "antibody" or "antibody peptide(s)" refer to an intact antibody, or a binding fragment thereof that competes with the intact antibody for specific binding. Binding fragments are produced by recombinant DNA techniques, or by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody substantially inhibits adhesion of a polypeptide to a specific binding partner when an excess of antibody reduces the quantity of the polypeptide bound to the specific binding partner by at least about 20%, 40%, 60% or 80%, and more usually greater than about 85% (as measured in an *in vitro* competitive binding assay).

The terms "chimeric protein" or "fusion protein" refer to a fusion of a first amino acid sequence encoding a polypeptide with a second amino acid sequence defining a domain (e.g., polypeptide portion) foreign to and not substantially homologous with any domain of the first polypeptide. A chimeric protein may present a foreign domain, which is found (albeit in a different protein) in an organism, which also expresses the first protein, or it may be an "interspecies", "intergenic", etc. fusion of protein structures expressed by different kinds of organisms.

The term "isolated" refers to material that is removed from its original environment. A cell is isolated if it is separated from some or all of the components that normally accompany it in its native state. For example, an "isolated population of cells," an "isolated source of cells," or "isolated platelets" and the like, as used herein, refer to in vitro or ex vivo separation of one or more cells from their natural cellular environment, and from association with other components of the tissue or blood, *i.e.,* it is not significantly associated with in vivo substances.

The terms "homology" and "identity" are used synonymously throughout and refer to sequence similarity between two peptides or between two nucleic acid molecules. Homology can be determined by comparing a position in each sequence, which may be aligned for purposes of comparison. When a position in the compared sequence is occupied by the same base or amino acid, then the molecules are homologous or identical at that position. A degree of homology or identity between sequences is a function of the number of matching or homologous positions shared by the sequences.

The terms "patient", "subject", "mammalian host", and the like are used interchangeably herein, and refer to mammals, including human and veterinary subjects.

As used herein, the terms "polynucleotide" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-, doublestranded and triple helical molecules, a gene or gene fragment, exons, introns, mRNA, tRNA, rRNA, ribozymes, antisense molecules, cDNA, recombinant polynucleotides, branched polynucleotides, aptamers, plasmids, vectors, isolated DNA of any sequence, isolated RNA of any sequence, nucleic acid probes, and primers. A nucleic acid molecule may also comprise modified nucleic acid molecules (e.g., comprising modified bases, sugars, and/or internucleotide linkers).

The term "polypeptide" is meant to refer to any polymer preferably consisting essentially of any of the 20 natural amino acids regardless of its size. Although the term "protein" is often used in reference to relatively large proteins, and "peptide" is often used in reference to small polypeptides, use of these terms in the field often overlaps. The term "polypeptide" refers generally to proteins, polypeptides, and peptides unless otherwise noted. Peptides described herein will be generally between about 0.1 to 100 KD or greater up to about 1000 KD, preferably between about 0.1, 0.2, 0.5, 1, 2, 5, 10, 20, 30 and 50 KD as judged by standard molecule sizing techniques such as centrifugation or SDS-polyacrylamide gel electrophoresis.

The phrases "parenteral administration" and "administered parenterally" refer to modes of administration other than enteral and topical administration, usually by injection, and includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, agent or other material other than directly into a specific tissue, organ, or region of the subject being treated (e.g., brain), such that it enters the animal's system and, thus, is subject to metabolism and other like processes, for example, subcutaneous administration.

The terms "prevent", "preventing", "prevention", "prophylactic treatment" and the like are meant to refer to reducing the probability of developing a disorder or condition in a subject, who does not have, but is at risk of or susceptible to developing a disorder or condition. Prevention and the like do not mean preventing a subject from ever getting the specific disease or disorder. Prevention may require the administration of multiple doses. Prevention can include the prevention of a recurrence of a disease in a subject for whom all disease symptoms were eliminated, or prevention of recurrence in a relapsing--remitting disease.

The term "single chain antibody" is meant to refer to an antibody based on a single chain format. Single chain antibodies can consist of the minimal binding subunit of antibodies. Single-chain antibodies can combine only those antigen-binding regions (e.g., all or some of the complement determining regions, CDRs present in the heavy chain variable region and/or the light chain variable region) of antibodies on a single stably-folded polypeptide chain. As such, single-chain antibodies are of considerably smaller size than classical immunoglobulins but retain the antigen-specific binding properties of antibodies. Single chain antibodies may be linked to a wide range of ligands, for example effector molecules or drug conjugates.

The term "soluble" as used herein is meant that a polypeptide, such as a fusion protein, that is not readily sedimented under low G-force centrifugation (*e.g.,* less than about 30,000 revolutions per minute in a standard centrifuge) from an aqueous buffer, *e.g.,* cell media. Further, the polypeptide is soluble if it remains in aqueous solution at a temperature greater than about 5-37°C and at or near neutral pH in the presence of low or no concentration of an anionic or non-ionic detergent. Under these conditions, a soluble polypeptide will often have a low sedimentation value, *e.g.,* less than about 10 to 50 svedberg units.

Aqueous solutions referenced herein typically have a buffering compound to establish pH, typically within a pH range of about 5-9, and an ionic strength range between about 2 mM and 500 mM. Sometimes a protease inhibitor or mild non-ionic detergent is added.

The terms "Fc domain" or "Fc region" is meant to refer to the immunoglobulin heavy chain "fragment crystallizable" region. Generally, an Fc domain is capable of interacting with a second Fc domain to form a dimeric complex. The Fc domain may be capable of binding cell surface receptors called Fc receptors and/or proteins of the complement system or may be modified to reduce or augment these binding activities. The Fc domain may be derived from IgG, IgA, IgD, IgM or IgE antibody isotypes and effect immune activity including opsonization, cell lysis, degranulation of mast cells, basophils, and eosinophils, and other Fc receptor-dependent processes; activation of the complement pathway; and protein stability *in vivo.*

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

"More than one" is understood as 2, 3, 4, 5, 6, 7, 8, 9, 10, 11, 12, 13, 14, 15, 16, 17, 18, 19, 20, 25, 30, 40, 50, 100, etc., or any value there between. "At least" a specific value, is understood to be that value and all values greater than that value.

Unless specifically stated or obvious from context, as used herein, the term "or" is understood to be inclusive.

Unless specifically stated or obvious from context, as used herein, the terms "a", "an", and "the" are understood to be singular or plural.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein can be modified by the term about.

This disclosure describes compositions and methods of preventing and/or treating hypercoagulation and/or thrombosis in a subject in need thereof. The methods include administering to the subject an amount of a composition that is effective to inhibit MRP-8/14 heterodimer and/or MRP-14 binding to platelet CD36. Inhibition of MRP-8/14 heterodimer and/or MRP-14 binding to platelet CD36 can inhibit hypercoagulation and/or thrombosis in the subject without adversely affecting and/or interfering with hemostasis or hemostatic parameter, such as bleeding time, platelet adhesion, or plasma coagulation activity *(e.g.,* aPTT and thrombin generation) in the subject.

As described in the Example, it was found that thrombotic occlusion was prolonged markedly in *Mrp14*^{*-*/*-*} mice, and thrombus formation was reduced in whole blood from *Mrp14*^{*-*/*-*} mice. MRP-14 and MRP-8/14 were found to be expressed in, and secreted by platelets. Infusion of wild platelets or purified *Mrp14*^{*-*/*-*} (or MRP-8/14) into *Mrp14*^{*-*/*-*} mice shortened the carotid artery occlusion time, indicating that platelet-derived MRP directly regulates thrombosis.

CD36 was identified as the platelet membrane receptor for MRP-14. MRP-14 was found to be capable of activating platelets in a CD36-dependent manner similar to oxidized LDL, as assessed by the phosphorylation of the known CD36 downstream signaling partners Vav and JNK. Agents that inhibit or reduce MRP-14 and/or MRP-8/14 binding to and/or activation of platelet CD36 can thus inhibit hypercoagulation and/or thrombus formation in blood of a subject in need thereof.

Embodiments described herein therefore relate to methods of preventing and/or treating hypercoagulation and/or thrombosis in a subject in need thereof by administering to the subject a composition that includes an agent, which specifically binds to or complexes with MRP-14, MRP-8/14 and/or CD36 to inhibit MRP-8/14 heterodimer and/or MRP-14 binding to platelet CD36 and inhibit hypercoagulation and/or thrombosis in the subject. The agent can bind to MRP-14, MRP-8/14 heterodimer, and/or CD36 to inhibit MRP-8/14 and/or MRP-14 induced activation of platelet CD36 signaling and hypercoagulation and/or thrombosis formation in a subject prone to or suffering from cardiovascular disease. The cardiovascular disease can include, for example, acute myocardial infarction, unstable angina, chronic stable angina, transient ischemic attacks, strokes, peripheral vascular disease, preeclampsia/eclampsia, deep venous thrombosis, embolism, disseminated intravascular coagulation and thrombotic cytopenic purpura, thrombotic and restenotic complications following invasive procedures resulting from angioplasty, carotid endarterectomy, post CABG (coronary artery bypass graft) surgery, vascular graft surgery, stent placements and insertion of endovascular devices and prostheses.

The agent (or therapeutic agent) that specifically binds to or complexes with MRP-14, MRP-8/14 and/or CD36 to inhibit MRP-8/14 heterodimer and/or MRP-14 binding to platelet CD36 can include any composition or substance that decreases and/or suppresses MRP-14 and/or MRP-8/14 activation of CD36. The agent can include a targeting small molecule, polypeptide, fusion protein, antibody, and/or a fragment of an antibody, such as an Fc fused to the extracellular segment of an Ig superfamily CAM (Fc chimera), that binds to and/or complexes with MRP-14, MRP-8/14 and/or CD36 and that can readily be administered to the subject using, for example, parenteral or systemic administration techniques (e.g., intravenous infusion).

The agent can include a soluble therapeutic polypeptide or fusion protein that binds to and/or complexes with MRP-14 and/or MRP-8/14 to inhibit MRP-8/14 heterodimer and/or MRP-14 binding to platelet CD36. The soluble polypeptide can have an amino acid sequence that is substantially homologous to consecutive amino acids (e.g., about 20 to about 400 consecutive amino acids) of a MRP-14 binding portion or domain of CD36. By substantially homologous, it is meant the polypeptide has at least about 80%, about 90%, about 95%, about 96%, about 97%, about 98%, about 99% or about 100% sequence identity with a portion of the amino acid sequence (e.g., about 10 to about 400 consecutive amino acids) of the MRP-14 binding portion of CD36.

In one example, the MRP-14 binding portion of CD36 can include, for example, the extracellular domain of CD36 and or a portion of the extracellular domain of CD36. Human CD36 can have an amino acid sequence of SEQ ID NO: 1. The extracellular domain of CD36 can have the amino acid sequence of SEQ ID NO: 2. The development of a CD36 polypeptide fragment or fusion protein that can target MRP-14 is based on a large body of structural and functional data. Specific MRP-14 binding portions of CD36 can be determined using solid phase binding techniques. For example, Klenotic et al., "Molecular Basis of Antiangiogenic Thrombospondin-1 Type 1 Repeat Domain Interactions with CD36", Arterioscler Thromb Vasc Biol. 2013;33:1655-1662 describes a series of bacterial GST CD36 fusions proteins encompassing the extracellular domain that can be used in solid phase binding studies to define the MRP-14 binding portion or site the extracellular domain of CD36 and to determine optimal concentration of inhibition. These smaller CD36 fusion proteins can include, for example, those spanning amino acids 93 to 120 of CD36 *(i.e.,* the CLESH domain (SEQ ID NO: 3)). As shown in the Example, it was found that MRP-14 bound to the CLESH domain containing peptide and hence a polypeptide that includes the CLESH domain (SEQ ID NO: 3) can be used to inhibit MRP-8/14 heterodimer and/or MRP-14 binding to platelet CD36

The polypeptide can have an amino acid sequence that is substantially homologous to about 10 to about 50 consecutive amino acids of a MRP-14 binding portion or domain of the extracellular domain of CD36 that is expressed by a platelet in the vasculature of a subject. For example, the MRP-14 binding portion of CD36 can include about 10 to about 50 consecutive amino acids of the amino acid sequence of SEQ ID NO: 2 and/or SEQ ID NO: 3.

The therapeutic polypeptides described herein can be subject to various changes, substitutions, insertions, and deletions where such changes provide for certain advantages in its use. In this regard, therapeutic polypeptides that bind to and/or complex with MRP-14 and/or MRP-8/14 to inhibit MRP-8/14 heterodimer and/or MRP-14 binding to platelet CD36 can correspond to or be substantially homologous with, rather than be identical to, the sequence of a recited polypeptide where one or more changes are made and it retains the ability to function as specifically binds to and/or complexes with MRP-14 and/or MRP-8/14.

The therapeutic polypeptide can be in any of a variety of forms of polypeptide derivatives and include, for example, amides, conjugates with proteins, cyclized polypeptides, polymerized polypeptides, analogs, fragments, chemically modified polypeptides, and the like derivatives.

The term "analog" includes any polypeptide having an amino acid residue sequence substantially identical to a sequence specifically shown herein in which one or more residues have been conservatively substituted with a functionally similar residue and that specifically binds to and/or complexes with MRP-14 and/or MRP-8/14 to inhibit MRP-8/14 heterodimer and/or MRP-14 binding to platelet CD36 as described herein. Examples of conservative substitutions include the substitution of one non-polar (hydrophobic) residue, such as isoleucine, valine, leucine or methionine for another, the substitution of one polar (hydrophilic) residue for another, such as between arginine and lysine, between glutamine and asparagine, between glycine and serine, the substitution of one basic residue, such as lysine, arginine or histidine for another, or the substitution of one acidic residue, such as aspartic acid or glutamic acid for another.

The phrase "conservative substitution" also includes the use of a chemically derivatized residue in place of a non-derivatized residue provided that such peptide displays the requisite binding activity.

"Chemical derivative" refers to a subject polypeptide having one or more residues chemically derivatized by reaction of a functional side group. Such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those polypeptides, which contain one or more naturally occurring amino acid derivatives of the twenty standard amino acids. For examples: 4-hydroxyproline may be substituted for proline; 5-hydroxylysine may be substituted for lysine; 3-methylhistidine may be substituted for histidine; homoserine may be substituted for serine; and ornithine may be substituted for lysine. Polypeptides described herein also include any polypeptide having one or more additions and/or deletions or residues relative to the sequence of a polypeptide whose sequence is shown herein, so long as the requisite activity is maintained.

The term "fragment" refers to any subject polypeptide having an amino acid residue sequence shorter than that of a polypeptide whose amino acid residue sequence is shown herein.

Additional residues may also be added at either terminus of a polypeptide for the purpose of providing a "linker" by which the polypeptides can be conveniently linked and/or affixed to other polypeptides, proteins, detectable moieties, labels, solid matrices, or carriers.

Amino acid residue linkers are usually at least one residue and can be 40 or more residues, more often 1 to 10 residues. Typical amino acid residues used for linking are glycine, tyrosine, cysteine, lysine, glutamic and aspartic acid, or the like. In addition, a subject polypeptide can differ by the sequence being modified by terminal-NH₂ acylation, *e.g.,* acetylation, or thioglycolic acid amidation, by terminal-carboxylamidation, *e.g.,* with ammonia, methylamine, and the like terminal modifications. Terminal modifications are useful, as is well known, to reduce susceptibility by proteinase digestion, and therefore serve to prolong half-life of the polypeptides in solutions, particularly biological fluids where proteases may be present. In this regard, polypeptide cyclization is also a useful terminal modification, and is particularly preferred also because of the stable structures formed by cyclization and in view of the biological activities observed for such cyclic peptides as described herein.

In some embodiments, the linker can be a flexible peptide linker that links the therapeutic peptide to other polypeptides, proteins, and/or molecules, such as detectable moieties, labels, solid matrices, or carriers. A flexible peptide linker can be about 20 or fewer amino acids in length. For example, a peptide linker can contain about 12 or fewer amino acid residues, *e.g.,* 3, 4, 5, 6, 7, 8, 9, 10, 11, and 12. In some cases, a peptide linker comprises two or more of the following amino acids: glycine, serine, alanine, and threonine.

Any polypeptide may also be used in the form of a pharmaceutically acceptable salt. Acids, which are capable of forming salts with the polypeptides, include inorganic acids such as trifluoroacetic acid (TFA) hydrochloric acid (HCl), hydrobromic acid, perchloric acid, nitric acid, thiocyanic acid, sulfuric acid, phosphoric acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid or the like.

Bases capable of forming salts with the polypeptides include inorganic bases such as sodium hydroxide, ammonium hydroxide, potassium hydroxide and the like; and organic bases such as mono-, di- and tri-alkyl and aryl-amines (e.g., triethylamine, diisopropylamine, methylamine, dimethylamine and the like) and optionally substituted ethanolamines (e.g., ethanolamine, diethanolamine and the like).

The therapeutic polypetides can be synthesized by any of the techniques that are known to those skilled in the polypeptide art, including recombinant DNA techniques. Synthetic chemistry techniques, such as a solid-phase Merrifield-type synthesis, can be used for reasons of purity, antigenic specificity, freedom from undesired side products, ease of production, and the like. A summary of the many techniques available can be found in, for example: Steward et al., "Solid Phase Peptide Synthesis", W. H. Freeman Co., San Francisco, 1969; Bodanszky, et al., "Peptide Synthesis", John Wiley & Sons, Second Edition, 1976; J. Meienhofer, "Hormonal Proteins and Peptides", Vol. 2, p. 46, Academic Press (New York), 1983; Merrifield, Adv. Enzymol., 32:221-96, 1969; Fields et al., int. J. Peptide Protein Res., 35:161-214, 1990; and U.S. Pat. No. 4,244,946 for solid phase peptide synthesis, and Schroder et al., "The Peptides", Vol. 1, Academic Press (New York), 1965 for classical solution synthesis. Appropriate protective groups usable in such synthesis are described in the above texts and in J. F. W. McOmie, "Protective Groups in Organic Chemistry", Plenum Press, New York, 1973.

In general, the solid-phase synthesis methods contemplated comprise the sequential addition of one or more amino acid residues or suitably protected amino acid residues to a growing polypeptide chain. Normally, either the amino or carboxyl group of the first amino acid residue is protected by a suitable, selectively removable protecting group. A different, selectively removable protecting group is utilized for amino acids containing a reactive side group such as lysine.

Using a solid phase synthesis as an example, the protected or derivatized amino acid can be attached to an inert solid support through its unprotected carboxyl or amino group. The protecting group of the amino or carboxyl group can then be selectively removed and the next amino acid in the sequence having the complimentary (amino or carboxyl) group suitably protected is admixed and reacted under conditions suitable for forming the amide linkage with the residue already attached to the solid support. The protecting group of the amino or carboxyl group can then be removed from this newly added amino acid residue, and the next amino acid (suitably protected) is then added, and so forth. After all the desired amino acids have been linked in the proper sequence, any remaining terminal and side group protecting groups (and solid support) can be removed sequentially or concurrently, to afford the final linear polypeptide.

It will be appreciated that the therapeutic polypeptides that bind to and/or complex with MRP-14 and/or MRP-8/14 to inhibit MRP-8/14 heterodimer and/or MRP-14 binding to platelet CD36 can be used as a starting point to develop higher affinity small molecules, antibodies, and/or antibody fragments with similar ligand binding capabilities. The development and screening of small molecules from pharmacophores of the polypeptides using, for example, *in silico* screening, can be readily performed, and the binding affinity of such identified molecules can be readily screened against the therapeutic polypeptides using assays described herein to select small molecule agents.

In other embodiments, the agent that binds to and/or complexes with MRP-14, MRP-8/14, and/or CD36 to inhibit MRP-14 and/or MRP-8/14 heterodimer binding to platelet CD36 can be an antibody, such as a monoclonal antibody, a polyclonal antibody, or a humanized antibody, that binds to and/or complexes with MRP-14, MRP-8/14, and/or CD36. The antibody can include Fc fragments, Fv fragments, single chain Fv (scFv) fragments, Fab' fragments, F(ab')2 fragments, single domain antibodies, camelized antibodies and other antibody fragments. The antibody can also include multivalent versions of the foregoing antibodies or fragments thereof including monospecific or bispecific antibodies, such as disulfide stabilized Fv fragments, scFv tandems ((scFv)₂ fragments), diabodies, tribodies or tetrabodies, which typically are covalently linked or otherwise stabilized *(i.e.,* leucine zipper or helix stabilized) scFv fragments; and receptor molecules, which naturally interact with a desired target molecule.

The antibody may be an antibody that has a single heavy chain variable region and no light chain sequence. Such an antibody, called a single domain antibody (sdAb) or a nanobody, has been reported to maintain the ability to bind to an antigen (Muyldemans S. et al., Protein Eng. (1994), 7 (9), 1129-35; and Hamers-Casterman C. et al., Nature (1993), 363 (6429), 446-8). These antibodies are also encompassed in the meaning of the functional fragment of the antibody as described herein.

Antibodies, such as monoclonal antibodies, a polyclonal antibodies, and humanized antibodies, that bind to and/or complex with MRP-14, MRP-8/14, and/or CD36 to inhibit MRP-14 and/or MRP-8/14 heterodimer binding to platelet CD36 can be provided by generating or acquiring antibodies to MRP-14 and/or CD36 and then screening those anti-MRP-14 or anti-CD36 antibodies that inhibit MRP-14 and/or MRP-8/14 heterodimer binding to platelet CD36 without adversely affect other ligand or agonist induced CD36 activation. Such antibodies can be screened in antibody blocking assays in which the anti-MRP-14 and/or anti-CD36 antibody binding affinity and ability to selectively block MRP-8/14 binding to platelet CD36 is measured.

In some embodiments, the antibody or antigen binding fragment thereof can specifically or selectively bind to MRP-14 and/or MRP-8/14 to block MRP-14 and/or MRP-8/14 binding with CD36. An example of an antibody that can specifically bind to MRP-14 and/or MRP-8/14 to block or inhibit MRP-14 and/or MRP-8/14 binding to platelet CD36 is 1H9(MA1-40222), which is commercially from Biolegend.

In other embodiments, the antibody or antigen binding fragment thereof can specifically or selectively bind to CD36 to block MRP-14 and/or MRP-8/14 binding with CD36. An example of an antibody that can specifically bind to CD36 to block or inhibit MRP-14 and/or MRP-8/14 binding to platelet CD36 is FA6-152(ab17044), which is commercially from Abcam.

In still other embodiments, the antibody can bind to the same epitope on MRP-14 and/or CD36 as the antibodies recited in this application (*e.g.,* 1H9 or FA6-152) or other antibodies that inhibit MRP-14 and/or MRP-8/14 heterodimer binding to platelet CD36. Such antibodies can be identified based on their ability to cross-compete with or competitively inhibit anti-MRP-14 blocking antibodies and/or anti-CD36 blocking antibodies in binding assays.

For example, an anti-MRP-14 antibody or antigen binding fragment thereof that competitively inhibits binding of an anti-MRP-14 antibody or antigen binding portion thereof can occur when 1H9 binds MRP-14 and this binding is inhibited by another antibody. Demonstration in an *in vitro* assay can translate the inhibitors effect *in vivo.* Therefore, any antibody or antigen binding portion thereof that shares the same epitope occupied by 1H9 (or FA6-152) can be used in the methods described herein. Thus all antibodies, small molecules, or any synthetic small or large molecules that competitively inhibit the binding of the antibodies described herein inhibit MRP-14 and/or MRP-8/14 heterodimer binding to platelet CD36.

Preparation of antibodies can be accomplished by any number of methods for generating antibodies. These methods typically include the step of immunization of animals, such as mice or rabbits, with a desired immunogen (*e.g.,* a desired target molecule or fragment thereof). Once the mammals have been immunized, and boosted one or more times with the desired immunogen(s), antibody-producing hybridomas may be prepared and screened according to well known methods. (See, for example, Kuby, Janis, Immunology, Third Edition, pp. 131-139, W.H. Freeman & Co. (1997), for a general overview of monoclonal antibody production,).

*In vitro* methods that combine antibody recognition and phage display techniques can also be used to allow one to amplify and select antibodies with very specific binding capabilities. See, for example, Holt, L. J. et al., "The Use of Recombinant Antibodies in Proteomics," Current Opinion in Biotechnology, 2000, 11:445-449,. These methods typically are much less cumbersome than preparation of hybridomas by traditional monoclonal antibody preparation methods.

In some embodiments, phage display technology may be used to generate an antibody or fragment thereof specific for a desired target molecule. An immune response to a selected immunogen is elicited in an animal (such as a mouse, rabbit, goat or other animal) and the response is boosted to expand the immunogen-specific B-cell population. Messenger RNA is isolated from those B-cells, or optionally a monoclonal or polyclonal hybridoma population. The mRNA is reverse-transcribed by known methods using either a poly-A primer or murine immunoglobulin-specific primer(s), typically specific to sequences adjacent to the desired V_{H} and V_{L} chains, to yield cDNA. The desired V_{H} and V_{L} chains are amplified by polymerase chain reaction (PCR) typically using V_{H} and V_{L} specific primer sets, and are ligated together, separated by a linker. V_{H} and V_{L} specific primer sets are commercially available, for instance from Stratagene, Inc. of La Jolla, Calif. Assembled V_{H}-linker-V_{L} product (encoding a scFv fragment) is selected for and amplified by PCR. Restriction sites are introduced into the ends of the V_{H}-linker-V_{L} product by PCR with primers including restriction sites and the scFv fragment is inserted into a suitable expression vector (typically a plasmid) for phage display. Other fragments, such as a Fab' fragment, may be cloned into phage display vectors for surface expression on phage particles. The phage may be any phage, such as lambda, but typically is a filamentous phage, such as Fd and M13, typically M13.

In phage display vectors, the V_{H}-linker-V_{L} sequence is cloned into a phage surface protein (for M13, the surface proteins g3p (pIII) or g8p, most typically g3p). Phage display systems also include phagemid systems, which are based on a phagemid plasmid vector containing the phage surface protein genes (for example, g3p and g8p of M13) and the phage origin of replication. To produce phage particles, cells containing the phagemid are rescued with helper phage providing the remaining proteins needed for the generation of phage. Only the phagemid vector is packaged in the resulting phage particles because replication of the phagemid is grossly favored over replication of the helper phage DNA. Phagemid packaging systems for production of antibodies are commercially available. One example of a commercially available phagemid packaging system that also permits production of soluble ScFv fragments in bacterial cells is the Recombinant Phage Antibody System (RPAS), commercially available from Amersham Pharmacia Biotech, Inc. of Piscataway, N.J. and the pSKAN Phagemid Display System, commercially available from MoBiTec, LLC of Marco Island, Fla. Phage display systems, their construction, and screening methods are described in detail in, among others, U.S. Patent Nos. 5,702,892, 5,750,373, 5,821,047 and 6,127,132.

In some embodiments, the therapeutic agent can include a polypeptide-Fc chimera that can specifically bind to and/or complex with MRP-14 and/or MRP-8/14 to inhibit MRP-8/14 heterodimer and/or MRP-14 binding to platelet CD36. Advantageously, The Fc region of the Fc chimera provides a binding site for other antibodies and can promote clustering, complexing, or aggregation of multiple antibodies, which can enhance the effectiveness of the polypeptide-Fc chimera in binding to and/or complexing with MRP-14 and/or MRP-8/14 to inhibit MRP-8/14 heterodimer and/or MRP-14 binding to platelet CD36.

Chimeric proteins that can combine the Fc regions of IgG with one or more domains of another protein, such as various cytokines and soluble receptors, are known. These chimeric proteins can be fusions of human Fc regions and human domains of another protein. These chimeric proteins would then be a "humanized Fc chimera", which would be advantageous as a human therapeutic. (See, for example, Capon et al., Nature, 337:525-531, 1989; Chamow et al., Trends Biotechnol., 14:52-60, (1996); U.S. Patent Nos. 5,116,964 and 5,541,087). The chimeric protein can be a homodimeric protein linked through cysteine residues in the hinge region of IgG Fc, resulting in a molecule similar to an IgG molecule without the C_{H1} domains and light chains. Due to the structural homology, such Fc fusion proteins exhibit *in vivo* pharmacokinetic profile comparable to that of human IgG with a similar isotype. This approach has been applied to several therapeutically important cytokines, such as IL-2 and IFN-α, and soluble receptors, such as TNF-Rc and IL-5-Rc (See, for example, U.S. Patent Nos. 5,349,053, 6,224,867 and 7,250,493).

In some embodiments, the polypeptide-Fc chimera is a chimeric molecule that includes a human sequence encoded polypeptide fused to a human Fc fragment and is capable of binding to or complexing with MRP-14 and/or MRP-8/14 to inhibit MRP-8/14 heterodimer and/or MRP-14 binding to platelet CD36.

The polypeptide portion of the polypeptide-Fc chimera used for methods described herein may be a polypeptide having an amino acid sequence that is substantially homologous to about 10 to about 50 consecutive amino acids of the MRP-14 binding portion of CD36 *(e.g.,* SEQ ID NO: 2).

The polypeptide portion of the polypeptide-Fc chimera, similar to the therapeutic polypeptide described above, can be subject to various changes, substitutions, insertions, and deletions where such changes provide for certain advantages in its use. In this regard, polypeptide portion correspond to or be substantially homologous with, rather than be identical to, the sequence of a recited polypeptide where one or more changes are made and it retains the ability to function as specifically binding to and/or complexing with MRP-14 and/or MRP-8/14 to inhibit MRP-8/14 heterodimer and/or MRP-14 binding to platelet CD36.

The Fc portion of the polypeptide-Fc chimera is a domain that binds an activating Fc receptor, such as an activating Fc Ig domain and includes the hinge region that allows for dimerization. The Fc portion of the polypeptide-Fc chimera can be readily adapted to render it species-specific. For use in a murine system, *e.g.,* cells derived from a mouse, the Fc fragment used to generate polypeptide-Fc can be that of a murine origin. In some embodiments, an Fc fragment of the murine IgG₂ₐ can be used.

In addition, it should be noted that when chimeric or fusion proteins with artificial sequences and activities are used as therapeutic agents, in some circumstances, patients treated with such a chimeric or fusion protein trigger an unwanted immune response, such as development of antibodies against the agent. Certain structural modifications of an Fc fragment have been shown to reduce immunogenicity of a therapeutic fusion protein. See, for example, U.S. Patent No. 6,992,174 B2 ; Liu et al., 2008, Immunological Reviews, 222:9-27. Such modifications may be useful for an effective design of the polypeptide-Fc chimera described herein.

The polypeptide-Fc chimera used in the methods may include a linking moiety that connects the polypeptide portion with an Fc fragment. In some cases, a hinge region of Fc fusion protein molecules serves as a spacer between the Fc region and the fused polypeptide (e.g., soluble receptor), allowing these two parts of the molecule to function separately.

In some embodiments, the Fc portion and the polypeptide portion that comprise a chimeric molecule are linked via a linking molecule which is not a contiguous portion of either the polypeptide or Fc portions and which covalently joins an amino acid of the polypeptide to an amino acid of Fc. As used herein, a linking molecule that is "not a contiguous portion" means that the polypeptide portion and the Fc portion of the chimera are connected via an additional element that is not a part of the polypeptide or immunoglobulin that is contiguous in nature with either of the chimeric portions and functions as a linker.

In some embodiments, the linking molecule may be a peptide linker. Where the linker is a peptide linker, the polypeptide-Fc chimera may be produced as a single recombinant polypeptide using a conventional molecular biological/recombinant DNA method.

In other embodiments, a flexible peptide linker can be used. A flexible peptide linker can be about 20 or fewer amino acids in length. For example, a peptide linker can contain about 12 or fewer amino acid residues, e.g., 3, 4, 5, 6, 7, 8, 9, 10, 11, and 12. In some cases, a peptide linker comprises two or more of the following amino acids: glycine, serine, alanine, and threonine.

Alternatively, a linking molecule may be a non-peptide linker. As used herein, a non-peptide linker useful for the method described herein is a biocompatible polymer including two or more repeating units linked to each other. Examples of the non-peptide polymer include but are not limited to: polyethylene glycol (PEG), polypropylene glycol (PPG), co-poly (ethylene/propylene) glycol, polyoxyethylene (POE), polyurethane, polyphosphazene, polysaccharides, dextran, polyvinyl alcohol, polyvinylpyrrolidones, polyvinyl ethyl ether, polyacryl amide, polyacrylate, polycyanoacrylates, lipid polymers, chitins, hyaluronic acid, and heparin. For more detailed descriptions of non-peptide linkers useful for Fc fusion molecules, see, for example, WO/2006/107124. Typically such linkers will have a range of molecular weight of about 1 kDa to 50 kDa, depending upon a particular linker. For example, a typical PEG has a molecular weight of about 1 to 5 kDa, and polyethylene glycol has a molecular weight of about 5 kDa to 50 kDa, and more preferably about 10 kDa to 40 kDa.

Molecular biological and biochemical techniques for preparing an Fc chimera are known. In some embodiments, the polypeptide-Fc chimera can be produced by conventional recombinatory DNA methods. In other embodiments, the polypeptide-Fc chimera can be produced as a single (*e.g.,* contiguous) recombinant polypeptide. In still other embodiments, two or more portions of the polypeptide-Fc can be produced as separate fragments and are subsequently linked together to yield the polypeptide-Fc chimera. For example, the polypeptide portion of the polypeptide-Fc chimera and an Fc portion of the polypeptide-Fc chimera can each be produced as separate recombinant polypeptides then fused together by a chemical linking means to yield the polypeptide-Fc. This production methodology may be preferred particularly in situations where a non-peptide linking molecule is employed. Similarly, this production methodology may be also preferred if a chimeric polypeptide-Fc does not fold correctly (*e.g.,* does not properly bind a ligand) when made as a single contiguous polypeptide.

For the production of recombinant polypeptides, a variety of host organisms may be used. Examples of hosts include, but are not limited to: bacteria, such as E. coli, yeast cells, insect cells, plant cells and mammalian cells. Choice of a host organism will depend on the particular application of the polypeptide-Fc chimera. The skilled artisan will understand how to take into consideration certain criteria in selecting a suitable host for producing the recombinant polypeptide. Factors affecting selection of a host include, for example, post-translational modifications, such as phosphorylation and glycosylation patterns, as well as technical factors, such as the general expected yield and the ease of purification. Host-specific post-translational modifications of the polypeptide-Fc chimera, which is to be used *in vivo,* should be carefully considered because certain post-translational modifications are known to be highly immunogenic (antigenic).

In some embodiments, the agents described herein can be provided in a pharmaceutical composition for administration to a subject in need thereof. The pharmaceutical compositions can include a pharmaceutically effective amount of a therapeutic agents described above and a pharmaceutically acceptable diluent or carrier.

The term "pharmaceutically acceptable carrier", "diluents", "adjuvant" and "physiologically acceptable vehicle" and the like are to be understood as referring to an acceptable carrier or adjuvant that may be administered to a patient, together with an agent, and which does not destroy the pharmacological activity thereof. Further, as used herein "pharmaceutically acceptable carrier" or "pharmaceutical carrier" are known in the art and include, but are not limited to, 0.01-0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

A "pharmaceutically effective amount" may be understood as an amount of the therapeutic agent that is effective to inhibit MRP-8/14 heterodimer and/or MRP-14 binding to platelet CD36 as well as inhibit hypercoagulation or thrombosis in a subject in need thereof.

Determination of a therapeutically effective amount is within the capability of those skilled in the art. The exact formulation, route of administration and dosage can be chosen by the individual physician in view of the patient's condition.

In some embodiments, the compositions or pharmaceutical compositions comprising the agents described herein can be used in methods of treating disease states in mammals which have disorders related to coagulation, such as in the treatment or prevention of unstable angina, refractory angina, myocardial infarction, transient ischemic attacks, thrombotic stroke, embolic stroke, disseminated intravascular coagulation including the treatment of septic shock, deep venous thrombosis in the prevention of pulmonary embolism or the treatment of reocclusion or restenosis of reperfused coronary arteries and/or vasculature. Further, these compositions are useful for the treatment or prophylaxis of those diseases which involve a number of thrombotic or thromboembolic events,

The term, "thrombotic or thromboembolic event," includes any disorder that involves a blockage or partial blockage of an artery or vein with a thrombosis or thromboembolism, all of which can be treated by the compositions described herein. A "thrombosis" is the formation of a clot (or thrombus) inside a blood vessel that can obstruct the flow of blood through the circulatory system. A "thromboembolism" involves formation in a blood vessel of a clot (thrombus) that breaks loose and is carried by the blood stream to lodge in another vessel area. The clot may lodge in a vessel in the lungs (pulmonary embolism), brain (stroke), gastrointestinal tract, kidneys, or leg. Thromboembolism is an important cause of morbidity (disease) and mortality (death), especially in adults.

A thrombotic or thromboembolic event occurs when a clot forms and lodges within a blood vessel. The clot may fully or partially block the blood vessel causing a thrombotic disorder such as a heart attack or stroke. Examples of thrombotic or thromboembolic events include thrombotic disorders such as acute myocardial infarction, unstable angina, ischemic stroke, acute coronary syndrome, pulmonary embolism, transient ischemic attack, thrombosis (*e.g.,* deep vein thrombosis, thrombotic occlusion and re-occlusion and peripheral vascular thrombosis) and thromboembolism. A thrombotic or thromboembolic event also includes first or subsequent thrombotic stroke, acute myocardial infarction, which occurs subsequent to a coronary intervention procedure, or thrombolytic therapy.

With respect to the venous vasculature, abnormal thrombus formation characterizes the condition observed in patients undergoing major surgery in the lower extremities or the abdominal area who often suffer from thrombus formation in the venous vasculature resulting in reduced blood flow to the affected extremity and a predisposition to pulmonary embolism. Abnormal thrombus formation further characterizes disseminated intravascular coagulopathy which commonly occurs within both vascular systems during septic shock, certain viral infections and cancer, a condition wherein there is rapid consumption of coagulation factors and systemic coagulation which results in the formation of life-threatening thrombi occurring throughout the microvasculature leading to widespread organ failure.

In some embodiments, the compositions described herein are useful in treating thromboembolic stroke, ischemic or hemorrhagic stroke, systemic embolism, stroke prevention in atrial fibrillation (SPAF), non-valvular atrial fibrillation, venous thromboembolism (VTE), prevention of VTE in knee or hip surgery, prevention of VTE in acute medically ill patients, and secondary prevention in acute coronary syndrome (ACS).

In some embodiments, the compositions are for treatment of embolic stroke, thrombotic stroke, venous thrombosis, deep venous thrombosis, acute coronary syndrome, or myocardial infarction.

In some embodiments, the compositions are for prevention of stroke in atrial fibrillation patients; prevention of thrombosis in medically ill patients; prevention and treatment of deep vein thrombosis; prevention of arterial thrombosis in acute coronary syndrome patients; and/or secondary prevention of myocardial infarction, stroke or other thrombotic events in patients who have had a prior event.

In some embodiments, the patient has atrial fibrillation. In some embodiments, the patient is a patient with non-valvular atrial fibrillation. In some embodiments, the patient has atrial flutter.

The compositions described herein can be administered *e.g*.*,* intravenously, parenterally, orally, subcutaneously, intramuscularly, transdermally (for example using an iontophoretic patch), intraocularly, intranasally, by inhalation, by implant, by suppository, or by other routes known to those skilled in the medical arts, taking into account the particular properties of the composition being administered and the particular therapy.

In one embodiment, the composition can be administered about 6 hours to 24 hours after thrombolysis has occurred, about 12 hours to 24 hours after thrombolysis has occurred, or about 20 hours to 24 hours after thrombolysis has occurred. In another aspect, the compound is administered multiple times. The number of doses administered will depend on the type and severity of the thrombotic or thromboembolic condition to be treated. This determination can be made by one skilled in the art and is within the scope of the invention.

In one embodiment, the compound may be administered on an ongoing basis to treat or prevent angina, myocardial infarction, stroke, pulmonary embolism, transient ischemic attack, coronary ischemic syndrome, Syndrome X, heart failure, diabetes, disorders in which a narrowing of at least one coronary artery occurs, thrombosis including catheter thrombosis, deep vein thrombosis, arterial vessel thrombosis, and peripheral vascular thrombosis, or thrombotic occlusion and re-occlusion, including re-occlusion subsequent to a coronary intervention procedure, or in connection with heart surgery or vascular surgery.

Therapeutically effective amounts of the agents are suitable for use in the compositions and methods described herein. The dosage regimen is selected in accordance with a variety of factors including type, species, age, weight, sex and medical condition of the patient; the severity of the condition to be treated; the route of administration; the renal and hepatic function of the patient; and the particular compound or salt or ester thereof employed.

Other embodiments described herein relate to a method of determining, predicting, or prognosticating whether a subject has an increased risk of thrombosis and/or cardiovascular disease activity by measuring the level of expressed or secreted MRP-14 and/or MRP-8/14 of platelets in and/or obtained from a subject. The major cause of acute coronary syndromes, including myocardial infarction and unstable angina, is plaque rupture and thrombosis. These ischemic events are typically precipitous without warning symptoms. Elevated platelet MRP-14 levels were found to be one of the strongest predictors of myocardial infarction in a subject with coronary artery disease as well as serve as an early and sensitive marker of myocardial necrosis. In some embodiments, the subjects risk of hypercoagulation, thrombosis, and/or cardiovascular disease activity (e.g., subjects who are at risk for the transition from "stable/chronic" cardiovascular disease, such as stable angina, to "unstable/acute" disease cardiovascular disease, such as acute coronary syndrome) can be determined by measuring the level of platelet MRP-14 and/or MRP-8/14 in blood or thrombus of the subject and correlating the measured level of MRP-14 and/or MRP-8/14 with an increased risk of hypercoagulation, thrombosis, and cardiovascular disease activity.

In some embodiments, the level of MRP-14 and/or MRP-8/14 can be measured by obtaining biological samples including platelets, such as blood samples or thrombus, from the subject using various well-known blood and platelet collection methods. In some embodiments, whole blood can be obtained from the subject and a platelet-rich plasma (PRP) can be prepared by centrifugation. Although gentler methods, such as density gradient centrifugation or electrophoresis can be used to isolate platelets. Samples containing the isolated platelets can be collected and stored using conventional techniques.

Methods of measuring the level of platelet MRP-14 and/or MRP-8/14 in the biological sample can include any existing, available or conventional separation, detection and quantification methods to measure the presence or absence (e.g., readout being present vs. absent; or detectable amount vs. undetectable amount) and/or quantity (e.g., readout being an absolute or relative quantity, such as, for example, absolute or relative concentration) of platelet MRP-14 and/or MRP-8/14 in blood or thrombus.

For example, such methods may include immunoassay methods, mass spectrometry analysis methods, or chromatography methods, or combinations thereof.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly an antibody, and the molecule(s) or analyte(s) of interest. Immunoassay technologies include without limitation direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1st ed., Humana Press 2000, ISBN 0896037282.

Generally, any mass spectrometric (MS) techniques that can obtain precise information on the mass of peptides, and preferably also on fragmentation and/or (partial) amino acid sequence of selected peptides (*e.g.,* in tandem mass spectrometry, MS/MS; or in post source decay, TOF MS), are useful herein. Suitable peptide MS and MS/MS techniques and systems are well-known per se (see, *e.g.,* Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. Detection and quantification of biomarkers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with the chromatographic and other methods described herein below.

Chromatography can also be used for measuring levels of MRP-14 and/or MRP-8/14 levels in a biological sample (*e.g.,* blood and/or plasma). As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, etc.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

In other embodiments, the level of platelet MRP-14 and/or MRP-8/14 can be measured in vivo using, for example, labeled MRP-8/14 probes that can be administered to the subject's vasculature. The labeled MRP-8/14 probes can include, for example, MRP-14 IgG-conjugated gadolinium nanoparticles that are described in Maiseyeu et al., "In Vivo Targeting of Inflammation-Associated Myeloid-Related Protein 8/14 Via Gadolinium Immunonanoparticles", Aterioscler Thromb Vasc Biol. 2012; 32(4): 962-970.

The measured level of platelet MRP-14 and/or MRP-8/14 in the biological sample (e.g., blood and/or plasma) or subject can be correlated to the presence of hypercoagulation, thrombosis, and/or cardiovascular disease activity in the subject, the increased likelihood or risk of hypercoagulation, thrombosis, and/or cardiovascular disease activity in the subject, and/or the severity of hypercoagulation, thrombosis, and/or cardiovascular disease activity in the subject by comparing the measured level with a reference or control level. An increase in the level of MRP-14 and/or MRP-8/14 compared to the control or reference level can be indicative of the presence, increased risk, and/or severity of hypercoagulation, thrombosis, and/or cardiovascular disease activity in the subject.

Distinct reference values may represent the prediction of a risk (*e.g.,* an abnormally elevated risk) of having a given disease or condition as taught herein vs. the prediction of no or normal risk of having said disease or condition. In another example, distinct reference values may represent predictions of differing degrees of risk of having such disease or condition.

In a further example, distinct reference values may represent the diagnosis of a given disease or condition as taught herein vs. the diagnosis of no such disease or condition (such as, *e.g.,* the diagnosis of healthy, or recovered from said disease or condition, etc.). In another example, distinct reference values may represent the diagnosis of such disease or condition of varying severity.

In yet another example, distinct reference values may represent a good prognosis for a given disease or condition as taught herein vs. a poor prognosis for said disease or condition. In a further example, distinct reference values may represent varyingly favorable or unfavorable prognoses for such disease or condition.

Such comparison may generally include any means to determine the presence or absence of at least one difference and optionally of the size of such different between values or profiles being compared. A comparison may include a visual inspection, an arithmetical or statistical comparison of measurements. Such statistical comparisons include, but are not limited to, applying a rule. If the values or biomarker profiles comprise at least one standard, the comparison to determine a difference in said values or biomarker profiles may also include measurements of these standards, such that measurements of the biomarker are correlated to measurements of the internal standards.

Reference values for the quantity of platelet MRP-14 and/or MRP-8/14 may be established according to known procedures previously employed for other biomarkers.

For example, a reference value of the quantity of platelet MRP-14 and/or MRP-8/14 indicative of the presence, increased risk, and/or severity of hypercoagulation, thrombosis, and/or cardiovascular disease activity in the subject may be established by determining the quantity of platelet MRP-14 and/or MRP-8/14 in sample(s) from one individual or from a population of individuals characterized by the particular diagnosis, prediction and/or prognosis of said disease or condition (*i.e.,* for whom said diagnosis, prediction and/or prognosis of hypercoagulation and/or thrombosis holds true).

Hence, by means of an illustrative example, reference values of the quantity of platelet MRP-14 and/or MRP-8/14 for the diagnoses of hypercoagulation, thrombosis, and/or cardiovascular disease activity vs. no hypercoagulation and/or thrombosis, and/or stable cardiovascular disease activity may be established by determining the quantity of platelet MRP-14 and/or MRP-8/14 in sample(s) from one individual or from a population of individuals diagnosed (e.g., based on other adequately conclusive means, such as, for example, clinical signs and symptoms, imaging, ECG, etc.) as, respectively, having or not having said disease or condition

In an embodiment, reference value(s) as intended herein may convey absolute quantities of platelet MRP-14 and/or MRP-8/14. In another embodiment, the quantity of platelet MRP-14 and/or MRP-8/14in a sample from a tested subject may be determined directly relative to the reference value (*e.g.,* in terms of increase or decrease, or fold-increase or fold-decrease). Advantageously, this may allow the comparison of the quantity of MRP-14 and/or MRP-8/14 in the sample from the subject with the reference value (in other words to measure the relative quantity of platelet MRP-14 and/or MRP-8/14 in the sample from the subject vis-a-vis the reference value) without the need first to determine the respective absolute quantities of platelet MRP-14 and/or MRP-8/14.

The expression level or presence of platelet MRP-14 and/or MRP-8/14 in a sample of a patient may sometimes fluctuate, *i.e.,* increase or decrease significantly without change (appearance of, worsening or improving of) symptoms. In such an event, the marker change precedes the change in symptoms and becomes a more sensitive measure than symptom change. Therapeutic intervention can be initiated earlier and be more effective than waiting for deteriorating symptoms. Early intervention at a more benign status may be carried out safely at home, which is a major improvement from treating seriously deteriorated patients in the emergency room.

Measuring the platelet MRP-14 and/or MRP-8/14 level of the same patient at different time points may in such a case thus enable the continuous monitoring of the status of the patient and may lead to prediction of worsening or improvement of the patient's condition with regard to a given disease or condition as taught herein. Alternatively, these reference values or ranges can be established through data sets of several patients with highly similar disease phenotypes, *e.g.,* from healthy subjects or subjects not having the disease or condition of interest. A sudden deviation of the platelet MRP-14 and/or MRP-8/14 levels from said reference value or range can predict the worsening of the condition of the patient *(e.g.,* at home or in the clinic) before the (often severe) symptoms actually can be felt or observed.

The various aspects and embodiments taught herein may further entail finding a deviation or no deviation between the quantity of platelet MRP-14 and/or MRP-8/14 measured in a sample from a subject and a given reference or control value.

A "deviation" of a first value from a second value may generally encompass any direction (*e.g.,* increase: first value>second value; or decrease: first value<second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

When a deviation is found between the quantity or level of platelet MRP-14 and/or MRP-8/14 measured in a sample from a subject and a reference value representing a certain diagnosis, prediction and/or prognosis of a given disease or condition as taught herein, said deviation is indicative of or may be attributed to the conclusion that the diagnosis, prediction and/or prognosis of said disease or condition in said subject is different from that represented by the reference value.

When no deviation is found between the quantity or level of platelet MRP-14 and/or MRP-8/14 measured in a sample from a subject and a reference value representing a certain diagnosis, prediction and/or prognosis of a given disease or condition as taught herein, the absence of such deviation is indicative of or may be attributed to the conclusion that the diagnosis, prediction and/or prognosis of said disease or condition in said subject is substantially the same as that represented by the reference value.

It is contemplated that one or more standards may be generated in which a normal level of platelet MRP-14 and/or MRP-8/14 in bodily sample of the subject is defined or identified. That standard may then be referred to as a way of determining whether platelet MRP-14 and/or MRP-8/14 levels in a given sample taken from an animal are normal or above-normal. The type of standard generated will depend upon the assay or test employed to evaluate the presence or level of platelet MRP-14 and/or MRP-8/14. In some embodiments, a score is assigned to a sample based on certain criteria and numbers within or above a certain number or range is deemed "above normal."

The level of MRP-14 and/or MRP-8/14 is considered above normal if an assay indicates that a particular measurement, amount or level is at about or at most about 80%, 75%, 70%, 65%, 60%, 55%, 50%,45%,40%, 35%, 30%, 25%, 20%, 15%, 10%, 5% or greater than the measurement, amount or level observed in samples that have normal levels of platelet MRP-14 and/or MRP-8/14. In other words, for example, a subject with normal platelet MRP-14 and/or MRP-8/14 levels exhibits a level of platelet MRP-14 and/or MRP-8/14 that is x; the sample from the subject being tested may be 1.5x, in which case, in some embodiments that subject's sample may be considered to have an above normal level of platelet MRP-14 and/or MRP-8/14.

Alternatively, the level of platelet MRP-14 and/or MRP-8/14 is considered above normal if an assay indicates that a particular measurement, amount or level is about or at least about 1, 2, 3, 4, 5, 6, 7, 8, 9, 10 or more standard deviations above the measurement, amount or level observed in subjects that have normal levels of platelet MRP-14 and/or MRP-8/14. In other cases, the level of platelet MRP-14 and/or MRP-8/14 may be considered above normal if a measurement, amount or level indicative of platelet MRP-14 and/or MRP-8/14 is or is at most 2, 3, 4, 5, 6, 7, 8, 9, 10, 15, 20, 25, 30, 35, 40, 45, 50 or more times more than the measurement, amount, or level indicative of platelet MRP-14 and/or MRP-8/14 in a normal subject.

In some embodiments, compositions including agents described herein can be administered to subject with measured elevated platelet MRP-14 and/or MRP-8/14 levels compared to control levels that are indicative of the presence, increased risk, and/or severity of hypercoagulation, thrombosis, and/or cardiovascular disease activity in the subject. Such subjects as described herein can be prone to or suffers from a cardiovascular disease, such as acute myocardial infarction, unstable angina, chronic stable angina, transient ischemic attacks, strokes, peripheral vascular disease, preeclampsia/eclampsia, deep venous thrombosis, embolism, disseminated intravascular coagulation and thrombotic cytopenic purpura, thrombotic and restenotic complications following invasive procedures resulting from angioplasty, carotid endarterectomy, post CABG (coronary artery bypass graft) surgery, vascular graft surgery, stent placements and insertion of endovascular devices and prostheses. The compositions can be administered to the subject at an amount effective to effective to inhibit MRP-8/14 and/or MRP-14 binding to platelet CD36 and inhibit hypercoagulation and/or thrombosis in the subject.

The following example is included to demonstrate different embodiments of the invention. It should be appreciated by those of skill in the art that the techniques disclosed in the examples, which follow represent techniques discovered by the inventor to function well in the practice of the claimed embodiments, and thus can be considered to constitute preferred modes for its practice.

### Example

In this Example, we show that platelet-derived MRP-14 directly modulates platelet function and thrombosis, without influence on tail bleeding time or other hemostatic parameters.

### METHODS

### Materials

Antibodies against human MRP-14, MRP-8, and MRP-8/14 were purchased from Abcam, BMA Biomedicals, and R&D Systems. Antibodies against mouse MRP-8 and MRP-14 were purchased from R&D Systems. Wug.E9 antibody against mouse P-selectin/CD62P conjugated with fluorescein isothiocyanate and JON/A (antibody against mouse-activated integrin GPIIb/IIIa) conjugated with R-phycoerythrin were purchased from emfret Analytics. Alexa Fluor secondary antibodies and Alexa Fluor 647-conjugated mouse anti-human CD42a were purchased from AbD Serotec. The following azide-free antibodies were used for the shear-induced platelet aggregation assays: control mouse IgG, anti-human CD36 (clone FA6-152), and TLR4 (clone HTA125) monoclonal antibodies from Abcam; anti-human MRP-14 (clone MRP1H9) from Biolegend; and anti-human RAGE (clone MAB11451) monoclonal antibodies from R&D Systems. Purified, recombinant human MRP-8 and MRP-14 were obtained from Novus Biologicals. Human α-thrombin was purchased from Haematologic Technologies and human fibrinogen from Enzyme Research Laboratories. Rose Bengal (4, 5, 6, 7-tetrachloro-3', 6-dihydroxy-2, 4, 5, 7-tetraiodospiro (isobenzofuran-1(3H), 9 [9H] xanthan)-3-1 dipotassium salt) was purchased from Sigma-Aldrich.

### Mice

*MRP14*^{*-*/*-*} mice were generated in the laboratory of Nancy Hogg, and *Cd36*^{*-*/*-*} mice were generated in the laboratory of Roy Silverstein. All mice had a congenic C57BL/6 background and were maintained in animal facilities at Case Western Reserve University School of Medicine. Eight- to 12-week-old male mice were used for all experiments.

### Platelet Isolation

Mouse platelets were prepared from the whole blood obtained by terminal inferior vena cava phlebotomy. Human platelets were prepared from the whole blood drawn from the antecubital vein of healthy volunteers after providing informed consent in accordance with University Hospitals Case Medical Center Institutional Review Board-approved protocol. Platelets were isolated, as described. Briefly, platelet-rich plasma was prepared by centrifugation (200g for 15 min for human; 2300g for 20 sec for mouse) of blood collected in 1:6 (v/v) acid citrate dextrose solution [2.5% (w/v) sodium citrate tribasic, 1.5% citric acid monohydrate, and 2.0% D-glucose]. Platelet-rich plasma was centrifuged (1000*g* for 10 min for human; 2300g for 3 min for mouse) and platelets suspended in Tyrode's buffer (130 mM sodium chloride, 5.0 mM potassium chloride, 1.0 mM magnesium chloride, 0.4 mM sodium phosphate, 5.0 mM D-glucose, 12 mM sodium bicarbonate, and 10 mM HEPES, pH 7.4). Platelet suspensions were adjusted to final density after counting particles >3-fl using a Z1 series Coulter Counter (Beckman Coulter, Fullerton, CA) equipped with a 50 µM aperture or were measured as part of a complete blood count (CBC) of sodium citrate anticoagulated mouse blood on a HEMAVET 950FS system in the Case Comprehensive Cancer Center, Case Western Reserve University School of Medicine.

### Activated partial thromboplastin time

The activated partial thromboplastin time (aPTT) was performed using Amelung KC4 coagulation analyzer (Sigma, St. Louis, MO), as described previously. Briefly, 100 µL of sodium citrate-anticoagulated plasma was incubated with 50 µL of a PTT reagent (Siemens, Washington DC) at 37°C for 5 minutes. 50 µL of 30 mM calcium chloride was then added and the time to clot formation was recorded.

### Thrombin generation

Tissue factor-induced thrombin generation time (TGT) was performed, as previously described. Briefly, a 1:2 dilution of mouse plasma in 25 mM HEPES, 175 mM NaCl2, containing 5 mg/mL bovine serum albumin, pH 7.7, was incubated with ∼3 pM tissue factor (3 µL of 1:60 dilution of stock Innovin, Siemens) and 0.42 mM Z-Gly-Gly-Arg-AMC. The reaction was initiated with the injection of 0.16 M calcium chloride, final concentration 16 mM. Substrate hydrolysis was measured on a fluorescent plate reader (NOVOstar, BMG Labtech). The TGT data are expressed as an arbitrary rate of fluorescent accumulation as determined by the second derivative of the raw fluorescent values. The lag time, peak height, and total area under the curve were calculated using Prism software (Graphpad, San Diego, CA).

### Photochemical carotid artery thrombosis

Male WT and *MRP-14*^{*-*/*-*} mice (age 7-9 weeks) were anesthetized by intraperitoneal injection with sodium pentobarbital (62.5mg/kg) and placed in the supine position on a dissecting microscope (Nikon SMZ-2T, Mager Scientific, Inc., Dexter, MI). A midline surgical incision was made to expose the right common carotid artery and a Doppler flow probe (MC 0.5PSL Nanoprobe, Model 0.5 VB, Transonic Systems, Ithaca, NY) was placed under the vessel. The probe was connected to a flowmeter (Transonic Systems Model TS420) and was interpreted with a computerized data acquisition program (Windaq, DATAQ Instruments, Arkron, OH). Rose Bengal at a concentration of 10 mg/mL in phosphate-buffered saline was then injected into the tail vein to administer a dose of 50 mg/kg. The mid portion of the common carotid artery was then illuminated with a 1.5-mW green light laser source (540 nm; Melles Griot, Carlsbad, CA) 5 cm from the artery. Blood flow was monitored continuously from the onset of injury. The time to occlusion, determined only after the vessel remained closed with a cessation of blood flow for 10 min, was recorded. In a separate group of animals, purified, recombinant human MRP-8, MRP-14, or MRP-8/14 (0.08 µg/g mouse or 0.4 µg/g mouse in 100 µL) was also infused into *MRP-14*^{*-*/*-*} mice via tail vein injection to determine the effect of extracellular MRP-8/14 on thrombosis.

### Laser-induced injury to the cremaster microcirculation using intravital microscopy

Thrombus formation *in vivo* after laser-induced injury to the arteriolar wall in the cremaster microcirculation of WT was compared with that of *MPP-14*^{*-*/*-*} mice using intravital microscopy (VIVO, 31 Inc.) was performed. Platelets were labeled in vivo using a FITC-conjugated rat anti-mouse CD41 antibody.

### Platelet α-Granule Release and GPIIb/IIIa Activation

Mouse platelets (5.0 X 105 in 20 µl of Tyrode's buffer containing 2.0 mM calcium chloride) were stimulated for 10 minutes at room temperature with agonist diluted in 5 µl Tyrode's buffer. The Wug.E9 and JON/A antibodies (5 µl each) were added to detect the expression of P-selectin (CD62P) and activated GPIIb/IIIa, respectively. After 20 minutes, platelets were fixed and diluted for FACS analysis by addition of 365 µl of 1% formaldehyde. Platelets were distinguished on the basis of side- and forward-light scatter, and the mean fluorescence intensity (MFI) of 10,000 platelets was measured per condition using FACSDiva LSRII (Becton Dickinson) and analyzed using Winlist.

### Platelet aggregation and secretion

*MRP14*^{*-*/*-*} and WT platelet-rich plasma was washed by centrifugation. Washed platelets were labeled with [¹⁴C] 5-hydroxytryptamine for 30 min at 37°C for dense granule secretion studies, as described previously. Briefly, at the conclusion of the incubation, the samples were treated with imipramine (2 µM). Washed platelets were incubated with collagen (2.5 µg/mL, BioData Corporation) or α-thrombin (2.5 nM, Hemetech) for aggregation studies in a Chronolog Model 440-VS dual channel aggregometer. Samples of activated platelets (0.2 mL) were removed and placed in a microcentrifuge tube containing 0.05 mL of 135 µM formaldehyde, 5 mM EDTA solution. After 5 min centrifugation at 12,000 g, the supernatants were collected for the degree of loss of the [¹⁴C] 5-hydroxytryptamine from the labeled platelets.

### Platelet adhesion

*Platelet adhesion assays* were performed as previously described. Briefly, 96 well plates were coated with 100 µL of GFOGER peptide (10 µg/mL) or vWF (30 µg/mL) overnight at 4°C. Washed platelets (1 x 10⁸/mL) in the presence of 1 mM Ca²⁺ on GFOGER or botrocetin (1 mg/mL) on vWF were incubated on the coated plates for 1 h at 37°C. After washing with PBS, adherent platelets were quantified based on their alkaline phosphatase activity

### Platelet spreading

*Platelet spreading assays* were performed as previously reported. Briefly, MatTek culture dish was coated with GFOGER (10 µg/mL) or vWF (30 µg/mL) overnight at 4°C. After blocking with 3 mg/mL BSA, washed platelets (3 x 10⁷/mL) were allowed to spread on GFOGER or vWF for 30 min at 37oC in the presence of Ca²⁺ (1 mM) or botrocetin (1 µg/ml), respectively. Adherent platelets were fixed with 3% paraformaldehyde, permeabilized with 0.1% Triton X-100, and stained with Alexa-Fluor 568-conjuaged phalloidin (25 µg/ml) for filamentous actin. Surface area was calculated in pixels using ImageJ software (NIH).

### Immunofluorescence microscopy

Coverslips were coated in 24-well plates with 0.5 ml fibrinogen (1mg/ml) for 24 h at 4°C and blocked with 5% bovine serum albumin (BSA) for 15 min at room temperature. Platelets (0.5 ml) adjusted to 1 X 10⁷ platelets/ml in Medium 199 were adhered to the coverslips at 37°C. After 30 min, an additional 0.5 ml of Medium 199 containing 2 nM α-thrombin *(i.e.,* final 1 nM α-thrombin), and platelets were cultured 6 h at 37°C. Platelets were fixed for 10 min by the addition of 1 ml of 8% formaldehyde *(i.e.,* final 4% formaldehyde), permeabilized for 20 min with 0.5% Triton X-100, and blocked with 1% BSA and 40 µg/ml non-immune human IgG. Primary and non-immune species-specific IgG control antibodies (2 µg/ml) were diluted in blocking solution and applied for at least 1 h at room temperature. The mouse monoclonal antibody to MRP-8 and MRP-14 (5.5, Abcam) was used for human platelets. Alexa Fluor 488-conjugated, species- and isotype-specific secondary antibodies (10 µg/ml) diluted in 1% BSA were applied in the dark for 1 h at room temperature. In some experiments, platelet GPIX was counterstained after extensive washing with Alexa Fluor 647-conjugated mouse anti-human CD42a. Coverslips were mounted on standard glass slides using Vecta shield mounting medium. Images were captured using a Leica microscope (DM2500) and captured with a RETIGA EXi Fast 1394 camera (QIMAGING, Surrey, BC, Canada).

### Immunoblotting

Protein samples were denatured by boiling in sodium dodecyl sulfate (SDS) sample buffer, run on 4% to 20% reducing SDS-polyacrylamide gel electrophoresis (PAGE), and transferred to nitrocellulose. The membrane was then blotted with indicated antibody, and the bands visualized with horseradish peroxidase-conjugated secondary antibody followed by the enhanced chemiluminescence Western blotting detection system (PerkinElmer Life and Analytical Sciences, Waltham, MA). Anti-tubulin mouse antibody was used as an internal control for protein loading.

### ELISA Assay

Human platelets (4.0 x 10⁸ platelets/mL) suspended in Tyrode's buffer and stimulated with 1.0 nM α-thrombin for 2 minutes. Following centrifugation at 10,000g for 10 min at 4°C, the supernatant was collected and stored at -20°C. The concentration of MRP-8/14 in the supernatant was determined by ELISA assay (Buhlmann Laboratories, Schonenbuch, Switzerland) according to the manufacturer's protocol.

### CD36 platelet signaling

Gel-filtered human platelets (2 x 10⁸/ml) containing 2 mM CalCl2 and 1 mM MgCl2 were incubated with 50 µg/mL oxLDL, 1 µg/mL MRP-14, or the combination for 10 min. Platelets were lysed with buffer containing protease and phosphatase inhibitors and lysates were then analyzed by immunoblot with anti-phospho-Vav (source) and phospho-JNK (source) antibodies. The membranes were then stripped and reprobed with antibodies to the total Vav (source) or JNK (source) protein and actin.

### Plate Binding Assay

High-protein binding plate (Nunc, Thermo Scientific) was coated with purified CD36 (Abcam) or BSA (10 µg/mL) overnight at 4°C. After blocking with 10% BSA at RT for 2 hours, purified human MRP-14 protein (0-2.5 µg/ml) was added and incubated for 2 hours at RT. After washing, plate was incubated with anti-human MRP-14 antibody for one hour followed by washing and incubation with HRP- conjugated secondary antibody that was detected with TMB substrate (Thermo Scientific) at 450nm.

### Histology and immunohistochemistry of tissue samples

At various time points following carotid artery photochemical injury, anesthesia was administered, the chest cavity opened, and the animals sacrificed by right atrial exsanguination. A 22-gauge butterfly catheter was inserted into the left ventricle for *in situ* pressure perfusion at 100 mm Hg with 0.9% saline for 1 min followed by fixation with 4% paraformaldehyde in 0.1 M phosphate buffer, pH 7.3, for 10 min. The carotid arteries were excised and immersed in buffered paraformaldehyde, embedded in paraffin, sectioned (5 µm), and stained with hematoxylin and eosin or Masson's trichrome. For immunohistochemistry, standard avidin-biotin procedures for mouse MRP-14 (R&D Systems) and mouse platelets (anti-GPIIb, BD Biosciences) were used. For each antibody, controls included species-specific non-immune IgG as well as omission of the primary antibody. A histologist blinded to genotype analyzed staining using a microscope equipped with a charge-coupled device camera (Zeiss AxioCam MRc5, Oberkochen, Germany) interfaced to a computer.

Human coronary artery thrombus was obtained using a thrombectomy catheter (Medtronic Export catheter) at the time of percutaneous coronary intervention for ST-segment elevation myocardial infarction prior to balloon angioplasty and stent deployment. Thrombus fragments were immersed in formalin, embedded in paraffin, sectioned (5 µm), and stained by immunofluorescence microscopy using the identical antibodies described for human platelets above.

### Mouse bleeding times

Tail bleeding times were measured by transecting the tails of anesthetized mice (50 mg/kg sodium pentobarbital) 5 mm from the tip, as previously described. Briefly, the transected tail tip was placed into a beaker containing saline at 37°C, and the time to complete cessation of bleeding for 30 seconds and 3 minutes was determined with a stopwatch.

### Thrombus formation under laminar flow conditions

The laminar flow chamber used in this assay has been described previously. Factor Xa inhibitor (Portola Pharmaceutical Inc.) anticoagulated blood was incubated with 0.2 µg/ml rhodamine-6G (Sigma-Aldrich) and then perfused for 5 minutes through human type III collagen-coated rectangular capillaries at 625 s-1, resulting in the deposition of adherent platelets and platelet aggregates. Thrombus formation under flow was then analyzed in real time and quantified by measuring fluorescence, thrombus area, and thrombus volume over time using computer-assisted imaging analysis (original magnification, ×40 and observation area, 360 × 270 µm).

### Adoptive transfer/platelet transfusion experiments

Blood from the inferior vena cava of 2 mice was collected directly into 3.8% sodium citrate (9:1 blood/ citrate ratio) and diluted with an equal amount of Tyrode's buffer. For isolation of platelets, anticoagulated and diluted blood was centrifuged to obtain platelet-rich plasma and then applied to a column packed with Sepharose 2B to obtain gel-filtered platelets. WT or *MRP14*^{*-*/*-*} platelets (1 × 108 in 250 µl) were injected into recipient *Mrp14*^{*-*/*-*} mice via the tail vein.

### Statistics

Data are presented as the mean ± SD. Comparisons between groups were performed by an unpaired, 2-tailed Student's t test. P values of less than 0.05 were considered statistically significant.

### Results

### Photochemical injury-induced arterial thrombosis is delayed in MRP14^{-/-} mice

To elucidate the effect of MRP-8/14 on the development of arterial thrombosis in real time, carotid arteries of WT and *Mrp14*^{*-*/*-*} mice were subjected to the Rose Bengal model of thrombosis, an endothelial cell photochemical injury model caused by local free-radical release. We then continuously monitored carotid artery blood flow with a vascular flow probe. Mean time to occlusive thrombus formation in WT mice was 27.1 ± 5.9 minutes and was significantly prolonged in *Mrp14*^{*-*/*-*} mice to 46.6 ± 22.9 minutes (*n* = 16 per group, P = 0.004) (Fig. 1A). We harvested carotid arteries 25 minutes after photochemical injury for histological analysis. Both perfusion and nonperfusion fixation techniques were used in order to visualize thrombus in situ. In nonperfused animals, a fibrin-platelet-rich thrombus with some red blood cells was evident in the lumen of WT arteries (Fig. 1B). In contrast, the lumen of *Mrp14*^{*-*/*-*} arteries was filled with blood at this 25-minute time point when the flow probe indicated that the vessel was widely patent. Nonocclusive thrombus (arrow) was visible along the wall of vessel. With perfusion fixation, we found that the occlusive thrombus within the lumen of injured WT arteries was still visible, whereas the lumen of *Mrp14*^{*-*/*-*} arteries was devoid of blood elements, indicating delayed thrombus formation and the instability of nonocclusive thrombus formed in *Mrp14*^{*-*/*-*} mice. Immunohistochemical analysis of serial sections of injured arteries from WT and *Mrp14*^{*-*/*-*} mice with anti-MRP-14 and the platelet specific anti-GPIIb antibodies showed positive MRP-14 staining that colocalized with platelets in WT arteries (Fig. 1C). We observed no staining for MRP-14 in *Mrp14*^{*-*/*-*} arteries.

### Impaired thrombus formation after laser-induced injury of the cremaster microvasculature in Mrp14^{-/-} mice

We used intravital microscopy to compare thrombus formation in vivo after laser-induced injury to the arteriolar wall in the cremaster microcirculation of *Mrp14*^{*-*/*-*} mice with that of WT mice. In WT mice, platelet accumulation in arterioles was evident within 30 seconds of laser injury (Fig. 1, D and E). In contrast, platelet accumulation was markedly attenuated in *Mrp14*^{*-*/*-*} mice (mean percentage of inhibition over time = 85.0% ± 5.1%, *n* = 10-15 arterioles per group). Initial platelet adhesion and small platelet aggregates were observed, but developing thrombi were unstable and embolized frequently.

### Platelet count and coagulation assays are similar in WT and Mrp14^{-/-} mice

Having observed delayed thrombosis in *Mrp14*^{*-*/*-*} mice, we set out to determine the mechanism by first performing screening platelet and coagulation assays in WT and *Mrp14*^{*-*/*-*} mice. The platelet count was similar in WT (736,000 ± 307,000 platelets/µl) and *Mrp14*^{*-*/*-*} (753,000 ± 241,000 platelets/µl, *P* = 0.90) mice. We assessed the coagulation activity of plasma using the activated partial thromboplastin time (aPTT) and a thrombin generation assay. The aPTT was not prolonged in *Mrp14*^{*-*/*-*}mice (WT: 66 ± 28 seconds versus *Mrp14*^{*-*/*-*}: 55 ± 16 seconds, *P* = 0.54). Tissue factor-induced total thrombin generation was similar in WT and *Mrp14*^{*-*/*-*} plasma (WT: 21,055 ± 407 versus *Mrp14*^{*-*/*-*}: 21,001 ± 4,041 arbitrary fluorescent units, *P* = 0.54). Moreover, multiple parameters of thrombin generation, including the lag time of thrombin generation, the maximum rate of thrombin generation, and the time to reach maximal thrombin activity, were comparable in WT and *Mrp14*^{*-*/*-*} mice (data not shown). Taken together, these data indicate that neither platelet count nor coagulation parameters likely account for delayed thrombosis in *Mrp14*^{*-*/*-*} mice.

### Platelets express MRP-8 and MRP-14 proteins

Although we have detected MRP-14 transcripts in human platelets, freshly-isolated human bone marrow megakaryocytes, and megakaryocytes generated *in vitro* by differentiation of human CD34-positive cells, we turned our attention to evaluating the expression of MRP-8 and MRP-14 protein in mouse platelets by Western blot analysis. Both MRP-8 and MRP-14 were detected in gel-filtered and washed platelets from WT, but not *Mrp14*^{*-*/}*⁻,* mice (Fig. 2A). To exclude the possibility that contaminating leukocytes were responsible for these S100 proteins, we also investigated MRP-8/14 expression using immunofluorescence and costaining for the platelet-specific marker glycoprotein IX (GPIX). Robust MRP-8/14 staining was evident in GPIX-positive human platelets (Fig. 2B). Finally, to further examine platelet MRP-8/14 expression, we performed 2-color flow cytometry on gel-filtered human platelets using platelet (anti-GPIIb/IIIa), leukocyte (anti-CD45), and anti-MRP-8/14 antibodies and observed abundant double-positive (GPIIb/ IIIa-positive/MRP-8/14-positive) platelets. Importantly, this staining was not accounted for on the basis of contaminating leukocytes, because MRP-8/14-positive cells were CD45 negative.

Next, we evaluated whether agonist stimulation is associated with increased surface expression and secretion of MRP-14. Thrombin stimulation of platelets was accompanied by increased platelet surface expression of MRP-14 (Fig. 2C). To verify secretion of MRP-14, gel-filtered human platelets were stimulated with thrombin, pelleted, and the supernatant was harvested for determination of MRP-8/14 concentration by ELISA. The concentration of MRP-8/14 in 1 ml of supernatant from 400 million thrombin-stimulated platelets was 1.03 ± 0.56 µg/ml.

To determine the relative abundance of MRP-8/14 compared with other intracellular platelet proteins/agonists, we assayed the protein content of human platelet MRP-8/14 compared with that of platelet CD40L, a platelet α-granule agonist that binds to GPIIb/IIIa and promotes thrombosis in an autocrine manner. We found that human platelet MRP-8/14 protein was more abundant than human platelet CD40L (Fig. 2D). To verify that platelet activation and thrombosis are associated with secretion of MRP-14, we hypothesized that MRP-14 levels would increase after carotid artery photochemical injury. Although the source of MRP- 14 is uncertain *(i.e.,* platelet, leukocyte, or endothelial cell derived), plasma MRP-14 levels increased 10 minutes after injury compared with levels in noninjured animals (Fig. 2E). In addition, when plasma and serum levels of MRP-8/14 in paired samples from 5 normal human donors were compared, we found that MRP-8/14 levels were significantly higher in serum than in plasma (Fig. 2F). Taken together, these observations indicate that platelets express both MRP-8 and MRP-14 protein and are capable of secreting MRP-8/14 after agonist stimulation and thrombus formation.

### Deficiency of MRP-14 attenuates platelet thrombus formation under flow ex vivo

Having demonstrated that thrombus formation is attenuated in *Mrp14*^{*-*/*-*} mice and that platelets express MRP-8/14, we next determined whether platelet MRP-14 itself regulates platelet function. We examined the role of MRP-14 in platelet thrombus formation under physiologic arterial shear conditions using a highly automated dynamic flow system. Thrombus formation was achieved by perfusion of anticoagulated blood labeled with rhodamine 6G through collagen-coated rectangular capillaries at an arterial shear rate of 625 s-1. We quantified the thrombus area and volume in real time using computer-assisted imaging analysis (Fig. 3A). Perfusion of blood resulted in the rapid formation of platelet thrombi that were significantly reduced in *Mrp14*^{*-*/*-*} compared with WT mice (percentage of inhibition of thrombus area = 49, *P* < 0.001; percentage of inhibition of thrombus volume = 60, *P* < 0.001) (Fig. 3, B and C).

Given the defect in platelet thrombus formation on collagen under flow, we assessed platelet activation by monitoring the expression of P-selectin and activated GPIIb/IIIa (JON/A-positive staining) in response to agonist stimulation. Washed platelets from WT and *Mrp14*^{*-*/*-*} mice were stimulated with collagen, thrombin, arachidonic acid, or ionomycin. P-selectin expression was significantly decreased in *Mrp14*^{*-*/*-*} compared with that in WT platelets following stimulation with collagen at 5 and 10 µg/ml (*P* = 0.005 and *P* = 0.048, respectively; Fig. 3D) and with 800 µM arachidonic acid (*P* = 0.027), but not with thrombin or ionomycin. Collagen-induced activation of GPIIb/IIIa was also significantly reduced (*P* = 0.010) in *Mrp14*^{*-*/*-*} versus WT platelets (Fig. 3E). Importantly, we verified that the expression levels of the platelet receptors GPIbα, GPVI, and α2β1 were comparable on WT and *Mrp4*^{*-*/*-*} platelets.

We also performed platelet secretion and aggregation studies to further characterize the nature of the *Mrp14*^{*-*/*-*} platelet defect. We assessed dense granule secretion by measuring agonist-induced secretion of [14C] 5-hydroxytryptamine and observed no significant difference in the uptake of [14C] 5-hydroxytryptamine between WT and *Mrp14*^{*-*/*-*} platelets (44% and 47%, respectively). After collagen stimulation, *Mrp14*^{*-*/*-*} platelets secreted 45% ± 19% of [14C] 5-hydroxytryptamine compared with 33% ± 3% for WT platelets (*P* = 0.32). Similarly, there was no difference in α-thrombin-induced platelet secretion (*Mrp14*^{*-*/*-*} platelets secreted 98% ± 1% of [14C] 5-hydroxytryptamine versus 95% ± 4% for WT platelets, *P* = 0.28). We monitored platelet aggregation during secretion experiments and found no difference in collagen- or thrombin-induced platelet aggregation between WT and *Mrp14*^{*-*/*-*} platelets. Finally, to determine whether MRP-14 modulates the platelet aggregation threshold, we evaluated ADP-induced fibrinogen binding by flow cytometry over a range of ADP concentrations and found that MRP-14 deficiency had no effect on ADP-stimulated fibrinogen binding.

### Hemostasis as determined by tail vein bleeding time is unimpaired in Mrp14^{-/-} mice

To assess the role of MRP-14 in hemostasis, we examined tail vein bleeding times. There was no difference in tail bleeding times between WT and *Mrp14*^{*-*/}*⁻mice* using complete cessation of bleeding for either 3 minutes or for 30 seconds as the criterion for bleeding time determination. Mean bleeding time for WT mice was 398 ± 200 seconds compared with 384 ± 218 seconds for *Mrp14*^{*-*/*-*} mice *(n* = 16 per group, *P* = 0.71) when complete absence of bleeding for 3 minutes was required (Fig. 4A). With the shorter bleeding cessation period of 30 seconds, the bleeding time in *Mrp14*^{*-*/*-*} mice was similar to that in WT mice (74 ± 26 seconds versus 79 ± 32 seconds, respectively, *P* = 0.64; Fig. 4B). We examined additional parameters of the primary hemostatic response, including platelet adhesion to the collagen peptide that binds α2β1 integrin GFOGER and platelet adhesion to and spreading on vWF. Platelet adhesion and spreading were similar in WT and *Mrp14*^{*-*/*-*} platelets.

### Transfusion of WT platelets shortens the prolonged time to carotid artery occlusion in Mrp14^{-/-} mice

Although we have provided evidence here that MRP-14 deficiency is associated with prolonged time to carotid artery occlusion after photochemical injury and with defects in collagen-stimulated platelet activation and shear-induced thrombus formation in collagen-coated capillaries, we used mice with global, rather than tissue-specific, deficiency of MRP-14, thereby limiting our ability to definitively conclude whether platelet-derived MRP-14 is critical for thrombus formation. To address this issue, we performed adoptive transfer or transfusion of WT and *Mrp14*^{*-*/*-*} donor platelets into *Mrp14*^{*-*/*-*} recipient mice prior to photochemical injury. *Mrp14*^{*-*/*-*} recipient mice that received *Mrp14*^{*-*/*-*} donor platelets formed occlusive thrombi within 44.0 ± 10.3 minutes (Fig. 5A). Strikingly, the time to occlusive thrombus formation was significantly shortened in *Mrp14*^{*-*/*-*} recipient mice receiving WT donor platelets to 30.3 ± 5.2 minutes (*P* = 0.02), nearly restoring the occlusion time to that of WT mice (27.1 ± 5.9 minutes, Fig. 1A).

### Role of intracellular versus extracellular MRP-8/14 in thrombosis

Transfusion of WT gel-filtered platelets nearly corrected the thrombotic defect in *Mrp14*^{*-*/*-*} mice, indicating that platelet MRP- 8/14 content regulates thrombosis (Fig. 5A). To determine whether extracellular MRP-8/14 action modulates thrombosis, we infused purified, recombinant human MRP-8, MRP-14, or MRP-8/14 (0.08 µg/g mouse) into *Mrp14*^{*-*/-} mice (Fig. 5B). Intravenous infusion of purified MRP-14 (30.9 ± 10.5 versus saline control infusion 46.4 ± 20.5 minutes, *P* = 0.025) or MRP- 8/14 (25.7 ± 13.3 versus saline control 46.4 ± 20.5 minutes, *P* = 0.007) into *Mrp14*^{*-*/*-*} mice shortened thrombotic occlusion time to that observed in WT mice (27.1 ± 5.9 minutes) (Fig. 5B). In contrast, infusion of purified MRP-8 alone had no significant effect on thrombotic occlusion time (MRP-8: 41.1 ± 24.4 minutes, *P* = 0.625), strongly suggesting that MRP-14 is responsible for thrombotic action of the MRP-8/14 heterodimer complex.

To verify the importance of extracellular MRP-8/14 in modulating platelet function, we assessed the effect of purified MRP-8/14 on platelet thrombus formation under flow conditions. Purified MRP-8/14 enhanced thrombus formation of *Mrp14*^{*-*/*-*} whole blood perfused through collagen-coated capillaries (Figs. 5, C and D). Finally, to extend these findings to human platelets, we examined the effect of targeting extracellular MRP-8/14 on platelet aggregate formation by assessing the effect of anti-MRP-14 monoclonal antibody on platelet thrombus formation under flow of anticoagulated human whole blood and found that anti-MRP-14 antibody inhibited platelet thrombus formation compared with control antibody (Fig. 5E).

### Identification of the candidate platelet receptor for MRP-14

Having observed that extracellular MRP-14 promotes thrombosis, we next sought to identify candidate platelet receptor(s). Putative receptors for MRP-8/14 on target cells include CD36, RAGE, and TLR4. Interestingly, both CD36 and RAGE signaling have been directly implicated in platelet activation and thrombosis. Platelets also express functional levels of toll-like receptor 4 (TLR4). We evaluated shear-induced platelet aggregation and thrombus formation in anticoagulated human whole blood in the presence of blocking antibodies against CD36, RAGE, or TLR4 (Fig. 6). Anti-CD36 monoclonal antibody inhibited shear-induced thrombus formation (percentage of inhibition = 53.8 ± 16.9, *P* = 0.012) (Fig. 6, A-C), and the extent of inhibition was comparable to that observed with anti-MRP-14 antibody (Fig. 5E). In contrast, blocking antibodies against either RAGE or TLR4 had no effect on platelet aggregate formation under these experimental conditions (Fig. 6, A-C).

Next, to determine whether CD36 is a candidate receptor for MRP-14, we examined the direct binding of MRP-14 to purified soluble CD36 in a plate binding assay. MRP-14 bound to soluble CD36-coated, but not BSA-coated, wells (Fig. 7A).

To establish whether CD36 is required for MRP-14 action, we crossed *Mrp14*^{*-*/*-*} mice with CD36-deficient (*Cd36*^{*-*/*-*}) mice to generate compound mutants (*Mrp14*^{*-*/}*⁻ Cd36*^{*-*/*-*}) and then subjected them to carotid photochemical injury. Deficiency of CD36 alone had no effect on thrombotic occlusion time (CD36: 23.1 ± 8.3 minutes versus WT: 27.1 ± 5.9 minutes, *P* = 0.167) in this model (Fig. 7B), a finding similar to that observed with the FeC13 carotid injury model using 12.5% FeCl3. Doubly deficient *Mrp14*^{*-*/}*⁻ Cd36*^{*-*/*-*} mice have prolonged thrombotic occlusion time that is no different than that in singly deficient *Mrp14*^{*-*/*-*} mice (44.7 ± 16.2 minutes versus 46.6 ± 22.9 minutes, respectively, *P* = 0.803; Fig. 7B). In direct contrast to experiments performed with *Mrp14*^{*-*/*-*} mice, in which infusion of purified MRP-14 (0.08 µg/g mouse) shortened the prolonged time to thrombotic occlusion (saline: 46.4 ± 20.5 minutes versus purified MRP-14: 30.9 ± 10.5 minutes, *P* = 0.026; Fig. 7B), infusion of MRP-14 into doubly deficient *Mrp14*^{*-*/}*⁻ Cd36*^{*-*/*-*} mice had no significant effect on the prolonged occlusion time (saline: 48.2 ± 15.3 minutes versus purified MRP-14: 47.2 ± 18.8 minutes, *P* = 0.884; Fig. 7B). Infusion of a 5-fold increased amount of MRP-14 (0.4 µg/g mouse weight) into *Mrp14*^{*-*/}*⁻ Cd36*^{*-*/*-*} mice also failed to shorten the prolonged occlusion time (43.0 ± 8.7 minutes, *P* = 0.40).

To verify the importance of CD36 in modulating platelet function in response to extracellular MRP-14, we assessed the effect of purified MRP-14 on platelet thrombus formation under flow using *Mrp14*^{*-*/}*⁻ Cd36*^{*-*/*-*} whole blood. While purified MRP-14 restored platelet thrombus formation of *Mrp14*^{*-*/*-*} whole blood perfused through collagen-coated capillaries (Figs. 7, C and D), purified MRP-14 had minimal enhancement of platelet thrombus formation of doubly deficient *Mrp14*^{*-*/}*⁻ Cd36*^{*-*/*-*} whole blood (Figs. 7, C and D). Further, we also examined whether MRP-14 is capable of activating platelets in a CD36-dependent manner. Oxidized LDL (oxLDL) initiates a CD36-mediated signaling cascade involving recruitment of Src family kinases (VAV, FYN, and LYN) and activation of JNK. Similarly to oxLDL, MRP-14 induces phosphorylation of VAV and JNK (Fig. 7, E and F). Since hyperlipidemia increases both plasma oxLDL and MRP-14 concentrations, we examined the phosphorylation of VAV and JNK after stimulating platelets with both oxLDL and MRP-14. Interestingly, MRP-14 potentiated oxLDL-induced phosphorylation of both VAV and JNK. Taken together, these observations indicate that platelet CD36 is required for MRP-14 action (Figs. 7, E and F).

### Platelet MRP-8/14 in human coronary thrombus

MI is most commonly caused by atherosclerotic plaque rupture and occlusive thrombus formation. To begin to examine the pathophysiological relevance of our findings of MRP-8/14 expression in platelets, we obtained coronary artery thrombi from patients (*n* = 4) presenting to the cardiac catheterization laboratory with acute STEMI. Angiography performed on one patient demonstrated thrombotic occlusion of the proximal right coronary artery (Fig. 8A) that was treated with aspiration thrombectomy followed by balloon angioplasty and stent deployment (Fig. 8B), resulting in a widely patent right coronary artery with no significant luminal narrowing (Fig. 8C). The thrombectomy catheter retrieved multiple coronary artery thrombi (Fig. 8D) that were then stained for platelets and MRP-8/14 using immunofluorescence microscopy. Platelet and MRP-8/14 staining were abundant and colocalized in this human coronary artery thrombus (Fig. 8, E-H). These findings were confirmed in the examination of intracoronary thrombi from three additional STEMI patients.

### The molecular domains responsible for MRP-14:CD36 binding and the downstream signaling that leads to platelet activation

We developed a simple, rapid solid-phase binding assay to quantify binding of recombinant, soluble CD36 to recombinant MRP-14 immobilized onto 96-well plastic plates. A large series of recombinant plasmids expressing CD36 peptides as fusion proteins with glutathione S-transferase (GST), maltose binding protein (MBP) and/or his-tag were developed. Conditions have been worked out to express mg amounts of the peptides in soluble form, including the entire extracellular domain and smaller peptides spanning amino acids 5-143, 5-93, 28-93, 67-157, 77-97, 93-120, 93-298, 118-182, 298-439, and 75-155. These reagents have been used to identify the domains in CD36 responsible for binding oxidized LDL as well as thrombospondins (TSP)-1 and -2 and other proteins containing the so-called thrombospondins type 2 repeat structural homology domain (TSR2). The TSR2-binding domain, which spans amino acids 93-120, was named the "CLESH" (CD36, LIMP-2, Emp Sequence Homology) domain (Fig. 9) because it is highly conserved in CD36 orthologs and homologs. We recently showed by NMR-based structural analyses, mutational studies, and in silica modeling approaches that TSP-1 interacts with CD36 CLESH through electrostatic interactions mediated by a positively charged TSR2 surface and a surface acidic cluster involving E101, D106, E108, and D109 in the CD36 CLESH domain.

To localize the CD36 binding site for MRP-14, recombinant CD36 peptides were employed in the solid phase MRP-14 binding assay to identify peptides that bind in a saturable manner with µM or less affinity. Controls included recombinant GST and non-binding CD36-GST peptides. Fig. 10 indicates that MRP-14 bound to the CLESH domain containing peptide (aa93-120) (SEQ ID NO: 3). Without wishing to be bound by theory, we believe that MRP-14-CLESH interactions mimic TSR2-CLESH interactions in that the surface acidic cluster within CLESH is likely to represent the binding site for a complementary cluster of residues that confer positive charge to the MRP-14 surface (K53, K59, K60, K63, K66).

The results of this Example identify a new pathway of thrombosis involving platelet MRP-14 and CD36 that does not affect bleeding time. This conclusion is supported by the following data: (a) the time to thrombotic occlusion was significantly prolonged in *Mrp14*^{*-*/*-*} mice; (b) laser-induced platelet thrombi in the cremaster microvasculature were reduced and less stable in *Mrp14*^{*-*/*-*} mice; (c) platelet thrombus formation under flow was reduced in whole blood from *Mrp14*^{*-*/*-*} mice; (d) MRP-8 and MRP-14 were expressed in platelets, and agonist stimulation led to increased platelet surface expression and secretion of MRP-8/14; (e) platelet count, aPTT, thrombin generation, and bleeding time were similar in WT and *Mrp14*^{*-*/*-*} mice; (f) transfusion of WT platelets or infusion of purified MRP-14 or MRP-8/14 into *Mrp14*^{*-*/*-*} mice shortened the prolonged carotid artery occlusion time in *Mrp14*^{*-*/*-*} mice; (g) compound deficiency of MRP-14 and CD36 resulted in a prolonged carotid artery occlusion time despite infusion of purified MRP-14; (h) MRP-14 was capable of activating platelets in a CD36-dependent manner *(i.e.,* induces phosphorylation of VAV and JNK); and (i) robust expression of MRP-8/14 was evident in platelet-rich coronary artery thrombi, causing acute MI.

MRP-8/14 complexes are also present in mouse cells, and extensive biochemical characterization has confirmed that mouse MRP-14 is functionally equivalent to its human counterpart. Analyses of mice that lack MRP-8 and MRP-14 have provided important insights into the function of these proteins. Although homozygous deletion of MRP-8 results in embryonic lethality, deletion of the *MRP14* gene does not affect viability and results in the additional loss of MRP-8 protein. Failure to produce mature MRP-8 protein in the presence of normal *MRP8* mRNA production likely results from an instability of MRP-8 in the absence of MRP-14. Thus, the *Mrp14*^{*-*/*-*} mice used in this and previous studies lack both MRP-8 and MRP-14 protein and MRP-8/14 complexes.

MRP-8/14 is known to be expressed by various cell types, including neutrophils, monocytes, tissue macrophages under conditions of chronic inflammation, mucosal epithelium, and involved epidermis in psoriasis. Immunofluorescence, immunoblotting, and ELISA data described herein indicate that platelets also express and secrete MRP- 8/14 protein. Immunofluorescence staining of purified platelets and coronary artery thrombi as well as flow cytometry of purified platelets indicate that MRP-8/14 protein is expressed in platelets. However, not all platelets appeared to express MRP-8/14. The basis for MRP-8/14 high versus low/negative platelet staining is unknown.

Our observations that transfusion of WT platelets or infusion of purified MRP-14 or MRP-8/14 into *Mrp14*^{*-*/*-*} mice shortened the prolonged carotid artery occlusion time of *Mrp14*^{*-*/*-*} mice show that extracellular, rather than intracellular, MRP-14 is largely responsible for MRP-14 action in thrombosis. In response to cytokines or during contact with activated endothelium, myeloid cells secrete heterodimeric MRP-8/14, which is the dominant extracellular form, through a tubulin-dependent "alternative" secretion pathway. Extracellular MRP-8/14 is then able to bind to receptors on target cells, including CD36, RAGE, TLR4, special carboxylated N-glycans, and heparin-like glycoaminoglycans.

Our findings that transfusion of WT platelets into *Mrp14*^{*-*/*-*} mice or infusion of purified MRP-14 into *Mrp14*^{*-*/*-*}, but not *Mrp14*^{*-*/}*⁻ Cd36*^{*-*/*-*} (Fig. 7B), mice shortened the prolonged carotid artery occlusion time in *Mrp14*^{*-*/*-*} mice show that platelet MRP-14 influences thrombosis secondary to secretion and binding to platelet CD36. Furthermore, while purified MRP-14 restored platelet thrombus formation in *Mrp14*^{*-*/*-*} whole blood perfused through collagen-coated capillaries (Fig. 7, C and D), purified MRP-14 had little effect on platelet thrombus formation in doubly deficient *Mrp14*^{*-*/}*⁻ Cd36*^{*-*/*-*} whole blood (Figs. 7, C and D). We performed a plate binding assay, which demonstrated that purified MRP-14 is capable of binding directly to soluble CD36 (Fig. 7A) and of activating platelets in a CD36-dependent manner, as indicated by the phosphorylation of VAV and JNK (Figs. 7, E and F).

The fact that the time to thrombotic occlusion after photochemical injury was prolonged in *Mrp14*^{*-*/*-*}, but not *Cd36*^{*-*/*-*}, mice raises the possibility that intracellular (e.g., cytoskeletal reorganization, calcium- coupled arachidonic acid signaling) as well as . extracellular MRP-14 action modulates platelet function. Indeed, a role for intracellular MRP-14 is supported by Fig. 7D, which shows that the addition of purified MRP-14 restored platelet thrombus formation in *Mrp14*^{*-*/*-*} whole blood perfused through collagen-coated capillaries to a level below that of WT whole blood. Its role is also supported by the finding that arachidonic acid-induced platelet activation was attenuated in *Mrp14*^{*-*/*-*} platelets.

Our studies show that MRP-14 deficiency did not interfere with tail bleeding time, platelet adhesion to and spreading on vWF or collagen, or plasma coagulation activity *(i.e.,* aPTT and thrombin generation). The identification of a new platelet-dependent pathway for thrombosis that does not affect hemostatic parameters, such as bleeding time and platelet adhesion and spreading, has clinical implications.

Finally, platelet MRP-8/14 can serve as a useful biomarker of coronary artery disease activity. The major causes of acute coronary syndromes, including MI and unstable angina, are plaque rupture and thrombosis. These ischemic events are typically precipitous and without warning symptoms. New approaches are required to identify patients who are at risk for the transition from "stable/chronic" to "unstable/acute" disease. Platelet MRP-8/14 expression is predictive of coronary artery disease activity *(i.e.,* stable angina versus acute coronary syndromes).

## Claims

1. A composition for use in inhibiting thrombosis in a subject having or at risk of thrombosis, the composition comprising an agent effective to inhibit MRP-8/14 binding to platelet CD36 and inhibit thrombosis in the subject,
wherein the agent binds to the MRP-8/14 heterodimer and inhibits MRP-8/14 induced activation of platelet CD36 signaling and/or thrombosis formation in the subject,
wherein the agent comprises an anti-MRP-14 antibody or antigen binding fragment thereof that inhibits binding of MRP-8/14 to CD36.

2. The composition for use of claim 1, wherein the agent inhibits thrombosis in the subject without inhibiting hemostasis.

## Patentansprüche

1. Zusammensetzung zur Verwendung bei der Hemmung von Thrombose bei einem Patienten mit Thrombose oder mit einem Risiko dafür, wobei die Zusammensetzung ein Mittel umfasst, das wirksam ist, um die Bindung von MRP-8/14 an das Blutplättchen CD36 zu hemmen und die Thrombose bei dem Patienten zu hemmen,
wobei das Mittel an das Heterodimer MRP-8/14 bindet und die MRP-8/14-induzierte Aktivierung das Blutplättchen-CD36-Signaling und/oder Thrombosebildung in dem Patienten hemmt,
wobei das Mittel einen Anti-MRP-14-Antikörper oder ein antigenbindendes Fragment davon umfasst, das die Bindung von MRP-8/14 an CD36 hemmt.

2. Zusammensetzung zur Verwendung nach Anspruch 1, wobei das Mittel eine Thrombose bei dem Patienten hemmt, ohne die Hämostase zu hemmen.

## Revendications

1. Composition pour une utilisation dans l'inhibition d'une thrombose chez un sujet ayant ou risquant d'avoir une thrombose, la composition comprenant un agent efficace pour inhiber la liaison de MRP-8/14 au CD36 plaquettaire et pour inhiber une thrombose chez le sujet,
dans laquelle l'agent se lie à l'hétérodimère de MRP-8/14 et inhibe l'activation induite par MRP-8/14 de la signalisation du CD36 plaquettaire et/ou la formation d'une thrombose chez le sujet,
dans lequel l'agent comprend un anticorps anti-MRP-14 ou un fragment se liant à un antigène de celui-ci qui inhibe la liaison de MRP-8/14 au CD36.

2. Composition pour une utilisation selon la revendication 1, dans laquelle l'agent inhibe une thrombose chez le sujet sans inhiber l'hémostase.


### SEQUENCE LISTING

<110> Simon, Daniel I.
<120> COMPOSITIONS AND METHODS OF TREATING THROMBOSIS
<130> CWR-023005WO ORD
<150> 61/913,263
   <151> 2013-12-07
<160> 3
<170> PatentIn version 3.5
<210> 1
   <211> 472
   <212> PRT
   <213> Homo sapiens
<400> 1
<210> 2
   <211> 435
   <212> PRT
   <213> Homo sapiens
<400> 2
   Arg Asn Cys Gly Leu Ile Ala Gly Ala Val Ile Gly Ala Val Leu Ala
   1 5 10 15
   Val Phe Gly Gly Ile Leu Met Pro Val Gly Asp Leu Leu Ile Gln Lys
   20 25 30
   Thr Ile Lys Lys Gln Val Val Leu Glu Glu Gly Thr Ile Ala Phe Lys
   35 40 45
   Asn Trp Val Lys Thr Gly Thr Glu Val Tyr Arg Gln Phe Trp Ile Phe
   50 55 60
   Asp Val Gln Asn Pro Gln Glu Val Met Met Asn Ser Ser Asn Ile Gln
   65 70 75 80
   Val Lys Gln Arg Gly Pro Tyr Thr Tyr Arg Val Arg Phe Leu Ala Lys
   85 90 95
   Glu Asn Val Thr Gln Asp Ala Glu Asp Asn Thr Val Ser Phe Leu Gln
   100 105 110
   Pro Asn Gly Ala Ile Phe Glu Pro Ser Leu Ser Val Gly Thr Glu Ala
   115 120 125
   Asp Asn Phe Thr Val Leu Asn Leu Ala Val Ala Ala Ala Ser His Ile
   130 135 140
   Tyr Gln Asn Gln Phe Val Gln Met Ile Leu Asn Ser Leu Ile Asn Lys
   145 150 155 160
   Ser Lys Ser Ser Met Phe Gln Val Arg Thr Leu Arg Glu Leu Leu Trp
                   165                 170                 175
   Gly Tyr Arg Asp Pro Phe Leu Ser Leu Val Pro Tyr Pro Val Thr Thr
               180                 185                 190
   Thr Val Gly Leu Phe Tyr Pro Tyr Asn Asn Thr Ala Asp Gly Val Tyr
           195                 200                 205
   Lys Val Phe Asn Gly Lys Asp Asn Ile Ser Lys Val Ala Ile Ile Asp
       210                 215                 220
   Thr Tyr Lys Gly Lys Arg Asn Leu Ser Tyr Trp Glu Ser His Cys Asp
   225                 230                 235                 240
   Met Ile Asn Gly Thr Asp Ala Ala Ser Phe Pro Pro Phe Val Glu Lys
                   245                 250                 255
   Ser Gln Val Leu Gln Phe Phe Ser Ser Asp Ile Cys Arg Ser Ile Tyr
               260                 265                 270
   Ala Val Phe Glu Ser Asp Val Asn Leu Lys Gly Ile Pro Val Tyr Arg
           275                 280                 285
   Phe Val Leu Pro Ser Lys Ala Phe Ala Ser Pro Val Glu Asn Pro Asp
       290                 295                 300
   Asn Tyr Cys Phe Cys Thr Glu Lys Ile Ile Ser Lys Asn Cys Thr Ser
   305                 310                 315                 320
   Tyr Gly Val Leu Asp Ile Ser Lys Cys Lys Glu Gly Arg Pro Val Tyr
                   325                 330                 335
   Ile Ser Leu Pro His Phe Leu Tyr Ala Ser Pro Asp Val Ser Glu Pro
               340                 345                 350
   Ile Asp Gly Leu Asn Pro Asn Glu Glu Glu His Arg Thr Tyr Leu Asp
           355                 360                 365
   Ile Glu Pro Ile Thr Gly Phe Thr Leu Gln Phe Ala Lys Arg Leu Gln
       370                 375                 380
   Val Asn Leu Leu Val Lys Pro Ser Glu Lys Ile Gln Val Leu Lys Asn
   385                 390                 395                 400
   Leu Lys Arg Asn Tyr Ile Val Pro Ile Leu Trp Leu Asn Glu Thr Gly
                   405                 410                 415
   Thr Ile Gly Asp Glu Lys Ala Asn Met Phe Arg Ser Gln Val Thr Gly
               420                 425                 430
   Lys Ile Asn
           435
<210> 3
   <211> 28
   <212> PRT
   <213> Homo sapiens
<400> 3
   Tyr Arg Val Arg Phe Leu Ala Lys Glu Asn Val Thr Gln Asp Ala Glu
   1               5                   10                  15
   Asp Asn Thr Val Ser Phe Leu Gln Pro Asn Gly Ala
               20                  25